(19) Europäisches Patentamt
European Patent Office
Office européen des brevets

(11) Veröffentlichungsnummer: **0 000 700**
**A1**

(12) # EUROPÄISCHE PATENTANMELDUNG

(21) Anmeldenummer: 78100325.6

(22) Anmeldetag: 07.07.78

(51) Int. Cl.²: C 07 D 211/00, C 07 D 401/00
C 07 D 491/00, C 08 K 5/00,
C 08 L 23/06

(30) Priorität: 15.07.77 CH 8794/77

(43) Veröffentlichungstag der Anmeldung: 21.02.79 Patentblatt 79/4

(84) Benannte Vertragsstaaten: BE CH DE FR GB NL

(71) Anmelder: CIBA-GEIGY AG
Patentabteilung Postfach
CH-4002 Basel(CH)

(72) Erfinder: Rasberger, Michael, Dr.
Waltersgrabenweg 6
CH-4125 Riehen(CH)

(72) Erfinder: Evans, Samuel, Dr.
Schützenrain 3
CH-4125 Riehen(CH)

(72) Erfinder: Moser, Paul, Dr.
Leimgrubenweg 56
CH-4125 Riehen(CH)

(54) **Metallkomplexe mit einem Phenolatgruppe enthaltenden chelatbindenden Anion; deren Herstellung und Verwendung als Lichtschutzmittel insbesondere in hochmolekularen Verbindungen.**

(57) Metallphenolate, welche mindestens eine am Stickstoff sterisch gehinderte Piperidinyl-gruppe besitzen eignen sich als Lichtschutzmittel für organisches Material, insbesondere für höhermolekulare Verbindungen.

EP 0 000 700 A1

Croydon Printing Company Ltd.

3-11251/S/+

BEZEICHNUNG GEÄNDERT
siehe Titelseite

## Neue Metallkomplexe

Die vorliegende Erfindung betrifft neue Metallkomplexe von zweifach oder dreifach geladenen Metallkationen mit einem Phenolatgruppen enthaltenden chelatbildenden Anion, deren Herstellung und Verwendung als Lichtschutzmittel in organischem Material, sowie das mit deren Hilfe geschützte organische Material.

In der DT-OS 2,625,967 sind Metallkomplexe mit sterisch gehinderten Aminen und einfach geladenen Anionen als Stabilisatoren für synthetische Polymere beschrieben worden. Es handelt sich dabei um Gemische von Metallsalzen und sterisch gehinderten Aminen.

Es wurden nun neue Metallchelatkomplexe gefunden, welche sich durch gute Lichtschutzwirkung und gute Extraktionsstabilität auszeichnen und eine gute Verträglichkeit in Polymeren besitzen.

Die neuen Metallkomplexe entsprechen der allgemeinen Formel I

$$\left[M^{q\oplus}\right] \left[L^{r\ominus}\right]_{(q-s)/r} \left[B^{\ominus}\right]_{s} \cdot m\,A \qquad (I)$$

worin

M ein zweifach oder dreifach positiv geladenes Metallion ist,

q 2 oder 3 ist, und

L ein 1 oder 2 Phenolatgruppen enthaltendes t-zähniges chelatbildendes Anion ist, welches mindestens eine Gruppe der Formeln IIa oder IIb

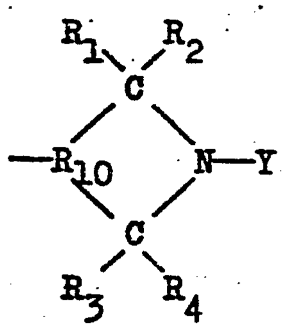 (IIa) 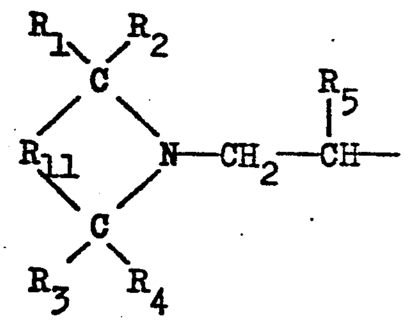 (IIb)

enthält, worin

t 2, 3 oder 4 ist, und

$R_1$,$R_2$,$R_3$ und $R_4$ unabhängig voneinander Alkyl sind, oder

$R_1$ und $R_3$ zusammen Alkylen sind, oder

$R_1$ und $R_2$ bzw. $R_3$ und $R_4$ unabhängig voneinander zusammen Alkylen oder Azaalkylen sind, und

$R_5$ Wasserstoff, Methyl oder Phenyl bedeutet, und

Y Wasserstoff, Oxyl, Alkyl, Alkenyl, Alkinyl, Alkoxyalkyl, Aralkyl, 2,3-Epoxypropyl, eine aliphatische Acylgruppe oder eine der Gruppen $-CH_2COOR_6$, $-CH_2-CH(R_7)-OR_8$, $-COOR_9$ oder $-CONHR_9$ ist, worin

$R_6$ Alkyl, Alkenyl, Phenyl, Aralkyl oder Cycloalkyl ist und

$R_7$ Wasserstoff, Methyl oder Phenyl ist, und

$R_8$ Wasserstoff, eine aliphatische oder aromatische, eine araliphatische oder alicyclische Acylgruppe bedeutet, worin der aromatische Teil gegebenenfalls mit Chlor, Alkyl, Alkoxy und/oder Hydroxy substituiert sein kann, und

$R_9$ Alkyl, Cyclohexyl, Phenyl oder Benzyl bedeutet, und

$R_{10}$ ein dreiwertiger und

$R_{11}$ ein zweiwertiger organischer Rest ist, wobei

$R_{10}$ und $R_{11}$ den N-haltigen Ring zu einem 5-, 6- oder 7-gliedrigen Ring ergänzen, und

r — 1 oder 2 und gleich der Anzahl koordinativ gebundener Phenolatanionen im Molekül ist, und

B — ein einfach negativ geladener Ligand ist, und

s — eine ganze Zahl von 0 bis 2 ist, wobei die Summe $(q-s)/_r + s = q$ sein muss, und

m — eine ganze Zahl von 0 bis 3 ist, wobei die Summe $t \cdot [(p-s)/_r] + s + m$ gleich der Koordinationszahl des Metallions M ist, wobei

A — $H_2O$ oder ein Amin der Formel III

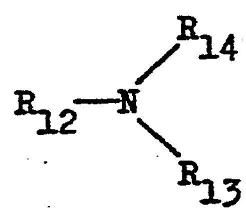

(III)

ist, worin

$R_{12}$ — gegebenenfalls substituiertes Alkyl, Cycloalkyl, Aryl, Aralkyl, eine gegebenenfalls substituierte Aminoalkylgruppe oder eine Piperidinylgruppe ist, und

$R_{13}$ — Wasserstoff oder gegebenenfalls substituiertes Alkyl, Cycloalkyl, eine gegebenenfalls substituierte Aminoalkylgruppe oder eine Piperidinylgruppe bedeutet, oder

$R_{12}$ und $R_{13}$ — zusammen mit dem N-Atom einen durch Alkylgruppen substituierten oder unsubstituierten Pyrrolidin-, Piperidin- oder Morpholinring bilden, und

$R_{14}$ — Wasserstoff oder gegebenenfalls substituiertes Alkyl bedeutet.

$R_1$, $R_2$, $R_3$ und $R_4$ sind als Alkyl unabhängig voneinander insbesondere Alkyl mit 1-6 C-Atomen, bevorzugt Aethyl und ganz besonders Methyl.

$R_1$ und $R_3$ sind zusammen als Alkylen insbesondere solches mit 1-6 C-Atomen, vor allem Methylen oder Aethylen.

$R_1$ und $R_2$ bzw. $R_3$ und $R_4$ sind zusammen als Alkylen insbesondere geradkettiges oder verzweigtes Alkylen mit 4-8 C-Atomen, vor allem Pentamethylen, und als Azaalkylen insbesondere geradkettiges oder verzweigtes Azaalkylen mit 4-16, vor allem 4-10 C-Atomen, das am N-Atom substituiert sein kann, insbesondere durch einen einwertigen Rest Y, vor allem durch Alkyl, wie solches mit 1-6 C-Atomen,insbesondere Methyl,das aber insbesondere am N-Atom unsubstituiert ist, wie 3-Aza-pentamethylen und insbesondere 2,2,4,4-Tetramethyl-3-aza-pentamethylen, 2,2,3,4,4-Pentamethyl-3-aza-pentamethylen oder das N-oxyl von 2,2,4,4-Tetramethyl-3-aza-pentamethylen. Bevorzugt ist nur eines der Paare $R_1/R_2$ und $R_3/R_4$ Alkylen oder Azaalkylen und das andere jeweils Alkyl, und insbesondere sind alle Reste $R_1$, $R_2$, $R_3$ und $R_4$ Alkyl. $R_5$ ist insbesondere Wasserstoff oder Methyl.

Y ist als Alkyl insbesondere solches mit 1-12 C-Atomen,bevorzugt α-unverzweigt, vor allem mit 1-4 C-Atomen und ganz besonders Methyl.

Y ist als Alkenyl insbesondere solches mit 3-12, vor allem mit 3-6 C-Atomen, wie Allyl, Methallyl, n-Hex-3-enyl, n-Oct-4-enyl und n-Undec-10-enyl.

Y ist als Alkinyl insbesondere solches mit 3-6 C-Atomen, wie Propargyl, n-But-1-inyl, But-2-inyl und n-Hex-1-inyl.

Bedeutet Y Alkoxyalkyl, so kann der Alkylteil 1-3 C-Atome enthalten und der Alkoxy-Teil aus 1-18 C-Atomen bestehen, wie z.B. in Methoxymethyl, Aethoxymethyl, 2-Methoxyäthyl, 2-Aethoxyäthyl, 2-n-Butoxyäthyl, 3-n-Butoxyäthyl, 2-Octoxyäthyl oder 2-Octadecyloxyäthyl. Insbesondere zu erwähnen sind Verbindungen, in denen Y eine Alkoxygruppe mit 2-5 C-Atomen bedeutet.

Y ist als Aralkyl ein solches mit 7-9 C-Atomen, wie z.B. Benzyl oder α-Phenyläthyl.

Y ist als aliphatische Acylgruppe insbesondere eine solche mit 1-4 C-Atomen, beispielsweise Formyl, Acetyl, Acryloyl oder Crotonyl, insbesondere Acetyl.

Ist Y die Gruppe $-CH_2COOR_6$, so bedeutet $R_6$ $C_1$-$C_{12}$ Alkyl, z.B. Methyl, Aethyl, Isopropyl, n-Butyl, Isobutyl, t-Butyl, Isopentyl, n-Octyl, n-Decyl oder n-Dodecyl; bevorzugt ist $R_6$ $C_1$-$C_4$ Alkyl oder $R_6$ bedeutet $C_3$-$C_6$ Alkenyl z.B. Allyl, 2-Butenyl oder 2-Hexenyl oder $C_7$-$C_8$ Aralkyl z.B. Benzyl oder α-Phenyläthyl oder schliesslich $C_5$-$C_7$ Cycloalkyl, insbesondere Cyclohexyl.

Ist Y die Gruppe $-CH_2-CH(R_7)-OR_8$, so bedeutet $R_7$ Wasserstoff, Methyl oder Phenyl, insbesondere Wasserstoff. $R_8$ ist als aliphatischer $C_1$-$C_{18}$ Acylrest beispielsweise Formyl, Acetyl, Propionyl, Butyryl, Octanoyl, Dodecanoyl, Stearoyl oder Acryloyl. $R_8$ ist als aromatischer $C_7$ Acylrest Benzoyl und als araliphatischer $C_8$-$C_9$ Acylrest, Cinnamoyl, Phenylacetyl oder Phenylpropionyl. Der aromatische Teil ist gegebenenfalls mit Chlor, $C_1$-$C_4$ Alkyl, wie Methyl, Aethyl, n-Propyl oder t-Butyl oder mit $C_1$-$C_8$ Alkoxy, wie Methoxy, Aethoxy, Butoxy oder Octoxy und/oder Hydroxy substituiert. Substituierte aromatische Acylgruppen sind z.B. Chlorobenzoyl, Toluoyl, Isopropylbenzoyl, 2,4-Dichlorobenzoyl, 4-Methoxybenzoyl, 3-Butoxybenzoyl, 2-Hydroxybenzoyl oder 3,5-Di-t.-butyl-4-hydroxy-benzoyl. Eine araliphatische substituierte Acylgruppe ist beispielsweise β-(3,5-Di-t.-butyl-4-hydroxyphenyl)-propionyl. Ist $R_8$ eine alicyclische $C_6$-$C_9$ Acylgruppe, so kann es sich um Cyclohexylcarbonyl oder 2,4-Dimethylcyclohexylcarbonyl handeln. Eine bevorzugte Bedeutung von $R_8$ ist auch Wasserstoff.

Ist Y die Gruppe $-COOR_9$, so ist $R_9$ $C_1$-$C_{12}$ Alkyl, z.B. Methyl, Aethyl, Isobutyl, t-Butyl, n-Hexyl, n-Octyl, n-Decyl oder n-Dodecyl. Bevorzugt sind als $R_9$ Alkylgruppen mit 1-4 C-Atomen.

Ist Y $-CONHR_9$, so ist $R_9$ insbesondere Cyclohexyl oder Phenyl.

Stellt A ein Amin der Formel III dar, so bedeuten dessen Substituenten $R_{12}$, $R_{13}$ und $R_{14}$ beispielsweise $C_1$-$C_{18}$ Alkyl, wie Methyl, Aethyl, iso-Propyl, sec-Butyl, n-Hexyl, n-Octyl, n-Decyl, n-Dodecyl oder n-Octadecyl, bevorzugt $C_1$-$C_8$ Alkyl.

Handelt es sich dabei um substituiertes Alkyl, so ist damit insbesondere $C_1$-$C_{18}$ Hydroxyalkyl gemeint, wie 1-Hydroxyäthyl, 1-Hydroxypropyl, 1-Hydroxy-1-methyl-äthyl, 3-Hydroxy-pentyl, oder 1-Hydroxy-2-methyl-äthyl, bevorzugt $C_1$-$C_6$ Hydroxyalkyl.

$R_{12}$ und $R_{13}$ können auch $C_5$-$C_{12}$ Cycloalkyl bedeuten, wobei es sich z.B. um Cyclopentyl, Cyclohexyl, 4-Methylcyclohexyl oder 4-tert.-Butylcyclohexyl handeln kann. $R_{12}$ kann auch die Bedeutung von $C_6$-$C_{18}$ Aryl haben, wobei es sich z.B. um Phenyl, Tolyl, Xylyl, tert.-Butylphenyl oder Dodecylphenyl handeln kann. $R_{12}$ als Aralkyl kann $C_7$-$C_{20}$ Aralkyl bedeuten und ist z.B. Benzyl, 4-Methylbenzyl, 4-t.-Butylbenzyl oder 4-Dodecylbenzyl, insbesondere Benzyl.

Bedeuten $R_{12}$ und $R_{13}$ eine gegebenenfalls substituierte Aminoalkylgruppe so handelt es sich insbesondere um eine mit einer Piperidinylgruppe substituierte Aminoalkylgruppe, z.B. der Formel IIc

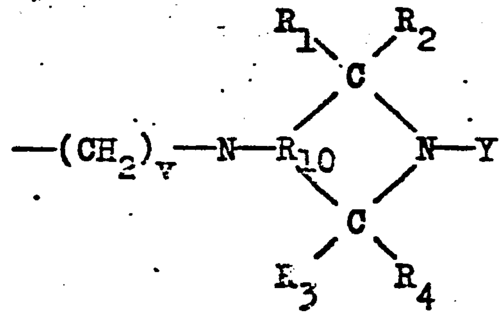

(IIc) ,

worin $R_1$, $R_2$, $R_3$, $R_4$, $R_{10}$ und Y oben angegebene Bedeutung haben und v eine ganze Zahl von 1 bis 8 ist.

Bedeuten $R_{12}$ und $R_{13}$ eine Piperidinylgruppe, so handelt es sich dabei vorzugsweise um eine solche der Formel (IIa), wobei $R_{10}$ ein dreiwertiger Rest $-CH_2-\overset{|}{C}H-CH_2-$ ist.

$R_{12}$ und $R_{13}$ können zusammen mit dem N-Atom des Liganden A einen durch Alkylgruppen substituierten oder unsubstituierten Pyrrolidin-, Piperidin- oder Morpholinring bilden. Es kann sich in diesem Fall beispielsweise um einen 2,5-Dimethylpyrrolidin-, 4-Methylpiperidin-, 2,2,6,6-Tetramethylpiperidin-, 2,6-Dimethylmorpholin-, Pyrrolidin-, Piperidin- oder Morpholinring handeln, insbesondere um einen 2,2,6,6-Tetramethylpiperidinring.

Der Ligand A stellt also definitionsgemäss ein primäres, sekundäres oder tertiäres Amin dar, das in der Lage ist, mit den oben angeführten Metallchelaten einen Komplex zu bilden. Beispiele für solche Amine sind: n-Butylamin, n-Dodecylamin, β-Aethylhexylamin, Benzylamin, 4-Octylbenzylamin, Dibutylamin, Dicyclohexylamin, Dioctadecylamin, Morpholin, 2,2,6,6-Tetramethylpiperidin, N-Aethylanilin, Tri-n-octylamin, N,N-Dimethylanilin, N,N-Dimethyl-cyclohexylamin, N-Aethylpiperidin, N-Methylpyrrolidin, Dibenzylpropylamin, N-Benzyl-2,5-dimethylpyrrolidin, 4-Dimethylamino-2,2,6,6-tetramethylpiperidin, 4-Dimethylamino-1,2,2,6,6-pentamethyl-piperidin, N,N'-Methyl-N,N'-(1,2,2,6,6-pentamethyl-4-piperidyl)-äthylendiamin, Hydroxyäthylamin, Di-(hydroxyäthyl)-amin, Tri-(hydroxyäthyl)-amin, Tri-(2-hydroxy-propyl)-amin, N-Phenyl-N,N-di-(hydroxyäthyl)-amin oder Hydroxypropyl-amin.

Die erfindungsgemässen Komplexe der Formel I enthalten pro Mol Metall $M^{q+}$ 0 bis 3 Mole des Aminliganden A, welcher ganz

oder teilweise durch Wasser ersetzt sein kann. Der in der Formel I mit m ausgedrückte Molanteil setzt sich also aus der Summe von m' Molen Aminligand und m'' Molen Wasser zusammen, wobei die durch m' und m'' ausgedrückten Molanteile keine ganze Zahl zu sein braucht. Bevorzugte Komplexe sind jene mit geringem Wassergehalt, da sich diese besser in unpolaren Polymeren lösen als stärker hydratisierte Komplexe. m' nimmt damit annähernd den Wert von m an.

Ein Kation M der Wertigkeit q, ist z.B. ein solches aus der Reihe $Mg^{2+}$, $Ca^{2+}$, $Sr^{2+}$, $Ba^{2+}$, $Zn^{2+}$, $Cd^{2+}$, $Al^{3+}$, $Sn^{2+}$, $Cr^{3+}$, $Co^{2+}$, $Ni^{2+}$, ein Oxokomplex von Metallionen, insbesondere $VO^{2+}$ und $MoO_2^{2+}$ oder ein Zinnalkylion der Formel $(R^*)_2Sn^{2+}$, oder $(CH_2CH_2COOR^*)_2Sn^{2+}$, worin R* ein $C_1$-$C_8$ Alkyl bedeutet, insbesondere jedoch Aethyl, n-Propyl, n-Octyl und vor allem n-Butyl, insbesondere $Ca^{2+}$, $Mg^{2+}$, $Zn^{2+}$, $Co^{2+}$, und vor allem $Ni^{2+}$ oder $Al^{3+}$. Die Koordinationszahlen dieser Kationen sind dem Fachmann bekannt, und betragen je nach Metall 4 oder 6.

q ist 2 oder 3, insbesondere 2.

r ist 1 oder 2 und entspricht der Anzahl Phenolatanionen im Liganden L. Enthält also ein Ligandmolekül mehrere phenolische -OH Gruppen, hängt es von den entsprechenden Stabilitätskonstanten und dem Verhältnis M/L ab, ob alle phenolische OH-Gruppen deprotoniert sind und damit zur Gesamtladung $r^-$ des Liganden L beitragen.

t gibt mit 2, 3 und 4 die Zähnigkeit des Liganden L an, d.h. dass t die Anzahl Donoratome im Molekül beschreibt, welche in unmittelbare Wechselwirkung zum Metallzentrum $M^{q+}$ treten. Es kommen 2-, 3- und 4-zähnige (dentate) Liganden für die gestellte Aufgabe in Frage, wobei die entstehenden Metallkomplexe ungeladen sind.

Der Chelatbildner L muss die Bedingung erfüllen, dass er mindestens eine der Gruppen IIa oder IIb enthält. Die Symbole $R_{10}$ und $R_{11}$ haben dabei eine Bedeutung, dass die Gruppen IIa und IIb beispielsweise Derivate des Pyrrolidins, Imidazolidins, 1,4-Diazacycloheptanons, Piperazins, 1-Aza-4-thiacyclohexans, Dipiperidyls und vor allem des Piperidins darstellen. Unter den zahlreichen Piperidinen sind nicht nur die einfachen in 4-Stellung substituierten Piperidine, sondern auch Spiro-Verbindungen wie 1-Oxa-3,8-diaza-spiro[4,5]-decane (Piperidinspirooxazolidine), 1-Oxa-6-aza-spiro[2,5]-octane (Piperidin-spiro-oxirane), 1,4-Dioxa-8-aza-spiro[4,5]-decan bzw. 1,5-Dioxa-9-azaspiro[5,5]-undecan, sowie die 1-, bzw. 4- analogen Thia- bzw. Aza-Verbindungen der beiden letztgenannten Piperidin-spiro-ketale, und vor allem 1,3,8-Triazaspiro[4,5]-decane (Piperidin-spirohydantoine) zu erwähnen.

Als besonders geeignet haben sich Metallkomplexe der Formel I erwiesen, in denen $L^{r-}$ ein 2-, 3- oder 4-zähniger Chelatbildner ausgewählt aus der Klasse bestehend aus den Gruppen IVa, IVb und IVc

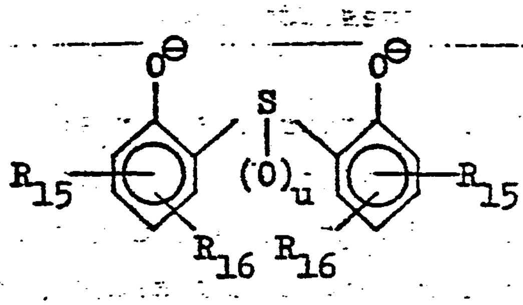

(IVa)

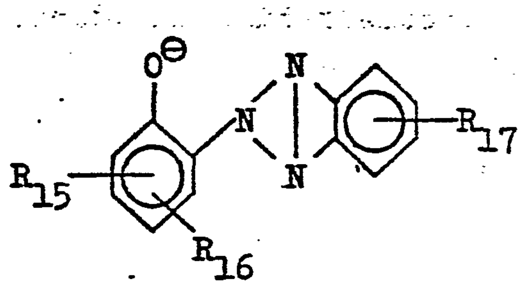

(IVb)

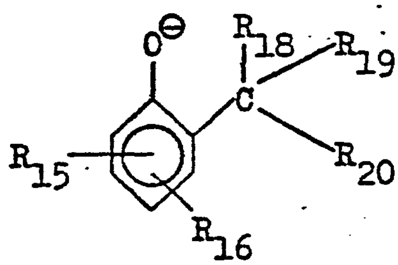

(IVc)

ist, worin

u 0, 1 oder 2 ist, und r oben angegebene Bedeutung hat, und

$R_{15}$ und $R_{16}$ unabhängig voneinander Wasserstoff, $C_1$-$C_{18}$ Alkyl, welches gegebenenfalls mit einer Gruppe Va, Vb oder Vc:

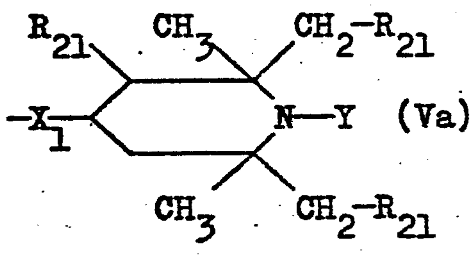 (Va), 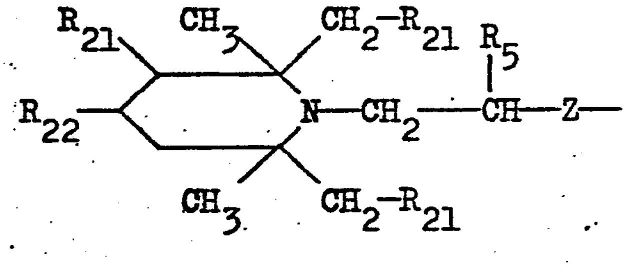 (Vb)

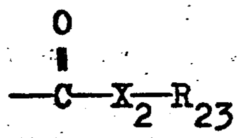 (Vc)

substituiert sein kann; oder eine Gruppe Vc,

oder

eine p-ständige Gruppe der Formel VI

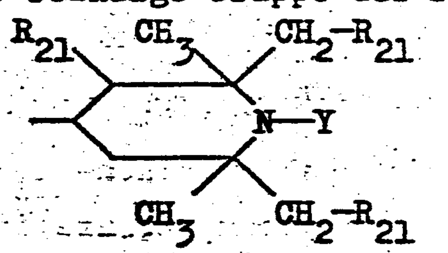 (VI) ,

Halogen oder einen Rest $-OR_{24}$ bedeuten, oder $R_{15}$ und $R_{16}$ zusammen einen 1,3-Butadien-1,4-diyl-Rest bilden, wobei

$R_5$ und Y die oben angegebene Bedeutung haben, und

$X_1$ und $X_2$ unabhängig voneinander -O- oder $-NR_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, wobei $X_1$ die oben angegebene Bedeutung hat, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, und

$R_{24}$ Wasserstoff, gegebenenfalls mit einer Gruppe der Formel Vc substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte, aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{17}$ Wasserstoff, $C_1$-$C_{18}$ Alkyl, Halogen, eine Gruppe $-OR_{24}$ oder eine Gruppe der Formel Vc bedeutet, und

$R_{18}$ eine Gruppe $-N(R_{25})R_{27}$ ist und

$R_{19}$ Wasserstoff ist, oder

$R_{18}$ und $R_{19}$ zusammen =O oder $=NR_{27}$ sind, und

$R_{20}$ Wasserstoff, $C_7$-$C_{14}$ Aralkyl, $C_7$-$C_{14}$ Alkylphenyl, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder, falls $R_{18}$ und $R_{19}$ zusammen =O sind eine Gruppe der Formel Va ist; oder $R_{20}$ ferner eine Gruppe der Formel VII

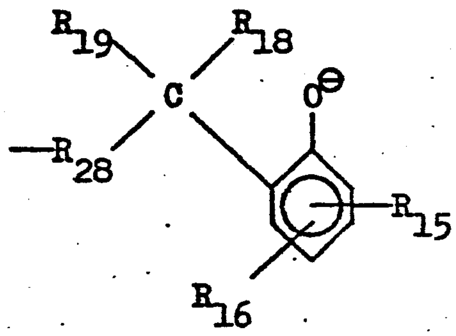

(VII) ,

bedeutet,

worin $R_{15}$, $R_{16}$, $R_{18}$ und $R_{19}$ oben angegebene Bedeutung haben, und

$R_{28}$ $C_1$-$C_{12}$ Alkylen, Butenylen, $C_6$-$C_{10}$ Arylen oder Diphenylen ist, bedeutet, und

$R_{27}$ Wasserstoff, $C_7$-$C_{14}$ Aralkyl, $C_7$-$C_{14}$ Alkylphenyl, Hydroxy, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder eine Gruppe der Formel VI, oder eine Gruppe der Formel

$-CH_2-CH(R_5)-OR_{26}$; oder $R_{27}$ auch eine Gruppe der Formeln VIIIa oder VIIIb

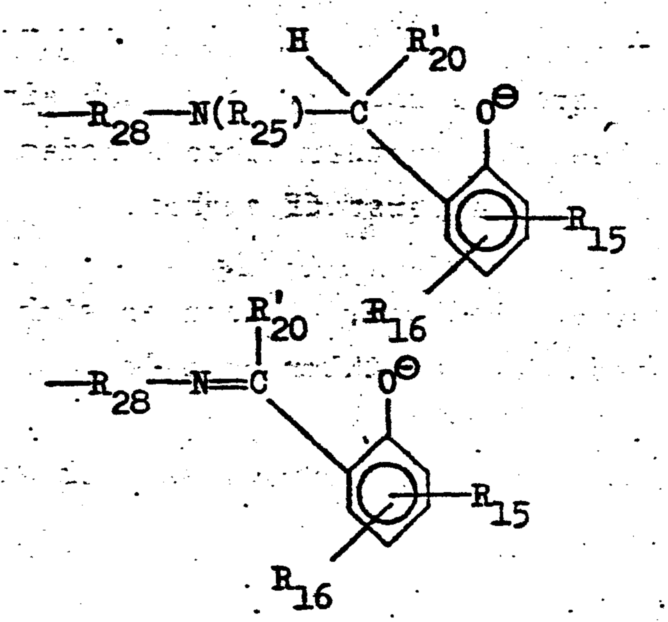

(VIIIa)

(VIIIb)

bedeutet, worin $R'_{20}$ die Bedeutung von $R_{20}$ mit Ausnahme der Gruppen der Formel VII hat, und $R_{15}$, $R_{16}$, $R_{25}$ und $R_{28}$ dieoben angegebene Bedeutung haben.

Die in den obenstehenden Formeln IVa, IVb, IVc, Va, Vb, Vc, VI, VII, VIIIa und VIIIb erwähnten Substituenten haben beispielhaft folgende Bedeutung:

$R_5$ und Y haben die oben erwähnte Bedeutung.

u bedeutet 0,1 oder 2, insbesondere 0.

Sind $R_{15}$ und $R_{16}$ unabhängig voneinander $C_1$-$C_{18}$ Alkyl, so kann es sich z.B. um Methyl, Aethyl, iso-Propyl, n-Butyl, t-Butyl, Amyl, n-Hexyl, n-Octyl, t-Octyl, n-Decyl, n-Dodecyl, n-Octadecyl oder 2-Aethyl-hexyl handeln. Bevorzugte Alkylgruppen sind solche mit 1-5 C-Atomen. Die Alkylgruppen können mit einer Gruppe der Formeln Va, Vb oder Vc, vorzugsweise Va oder Vc substituiert sein; es werden dabei Alkylgruppen mit 1-4 C-Atomen bevorzugt. Bevorzugte Verbindungen der Formel I sind solche, in welchen höchstens einer der Reste $R_{15}$ und $R_{16}$ mit einer Gruppe der Formeln Va, Vb oder Vc substituiert ist. Die Reste $R_{15}$ oder $R_{16}$ können auch eine Gruppe Vc sein.

Einer der Reste $R_{15}$ und $R_{16}$ kann auch eine Gruppe der Formel VI darstellen. Aus synthetischen Gründen muss diese in p-Stellung zum Phenolatsauerstoff stehen.

Ist $R_{15}$ und $R_{16}$ Halogen, so kann es sich dabei um Chlor oder Brom, insbesondere um Chlor handeln.

$R_{15}$ und $R_{16}$ bedeuten vorzugsweise auch Wasserstoff oder $-OR_{24}$.

$R_{17}$ ist als $C_1$-$C_{18}$ Alkyl z.B. Methyl, Aethyl, Propyl, Butyl, n-Hexyl, n-Octyl, n-Decyl, n-Dodecyl oder n-Octadecyl, bevorzugt sind Alkylgruppen mit 1-12 C-Atomen.

$R_{17}$ ist als Halogen insbesondere Chlor.

Bevorzugt sind auch Verbindungen, in denen $R_{17}$ Wasserstoff, $-OR_{24}$ und insbesondere eine Gruppe der Formel Vc bedeutet.

Bedeuten $R_{20}$ und $R_{27}$ $C_7$-$C_{14}$ Aralkyl, so kann es sich beispielsweise um Benzyl, α-Phenyläthyl oder 2-Phenylpropyl, insbesondere um Benzyl handeln.

Sind $R_{20}$, $R_{24}$, $R_{25}$ und $R_{27}$ $C_1$-$C_{18}$ Alkyl, so sind beispielsweise Methyl, Aethyl, iso-Propyl, n-Butyl, t-Butyl, Amyl, n-Hexyl, n-Octyl, t-Octyl, n-Decyl, n-Dodecyl, oder n-Octadecyl gemeint. Von diesen sind Alkylgruppen mit 1-12 C-Atomen bevorzugt. Bevorzugt sind auch Alkylgruppen, welche mit einer Gruppe der Formel Vc substituiert sind, darunter vor allem solche mit 1-4 C-Atomen.

$R_{20}$, $R_{24}$ und $R_{27}$ sind als $C_2$-$C_{12}$ Alkenyl, z.B. Allyl, Methallyl, n-Hex-3-enyl, n-Oct-4-enyl oder n-Undec-10-enyl. Bevorzugt werden Alkenylgruppen mit 2-6 C-Atomen, welche gegebenenfalls mit einer Gruppe der Formel Vc substituiert sein können.

$R_{20}$ und $R_{27}$ können auch $C_5$-$C_{12}$ Cycloalkyl bedeuten. Es handelt sich dann z.B. um Cyclopentyl, Cyclohexyl, Methylcyclohexyl, Cyclooctyl, Dimethylcyclohexyl, Propylcyclohexyl oder Dehydronaphthyl-α-methyl; insbesondere um Cyclohexyl. Sind $R_{20}$ und $R_{27}$ Cycloalkyl, so sind sie vorzugsweise mit einer Gruppe der Formel Vc substituiert.

Bedeuten $R_{20}$, $R_{24}$ und $R_{27}$ $C_6$-$C_{14}$ Aryl, so ist damit z.B. Alkylphenyl mit 1-8 C-Atomen, insbesondere mit 1-4 C-Atomen im Alkylteil, besonders aber Phenyl gemeint. Der Arylring ist vorzugsweise mit einer Gruppe der Formel Vc substituiert.

Bevorzugt sind ausserdem Verbindungen in denen eines von $R_{20}$ und $R_{27}$ Wasserstoff ist.

$R_{27}$ kann bevorzugt eine Gruppe der Formel VI bedeuten.

$R_{21}$ ist als $C_1$-$C_8$ Alkyl z.B. Methyl, Aethyl, iso-Propyl, n-Butyl, Amyl, n-Hexyl oder n-Octyl. Bevorzugt sind Alkylgruppen mit 1-4 C-Atomen, insbesondere ist $R_{21}$ jedoch Methyl oder Wasserstoff.

$R_{23}$ und $R_{26}$ sind als $C_1$-$C_{18}$ Alkyl beispielsweise Methyl, Aethyl, iso-Propyl, n-Butyl, t-Butyl, Amyl, n-Hexyl, n-Octyl, t-Octyl, n-Decyl, n-Dodecyl, n-Octadecyl.

$R_{23}$, $R_{25}$ und $R_{26}$ sind als $C_3$-$C_6$ Alkenyl z.B. Allyl, Methallyl, But-2-enyl, Hex-3-enyl, insbesondere Allyl.

Bedeuten $R_{23}$ und $R_{25}$ $C_3$-$C_4$ Alkinyl, so handelt es sich insbesondere um Propargyl.

$R_{23}$, $R_{24}$ und $R_{25}$ sind als $C_5$-$C_7$ Cycloalkyl beispielsweise Cyclopentyl, Cyclohexyl oder Methylcyclohexyl, insbesondere Cyclohexyl.

$R_{23}$ und $R_{25}$ sind als $C_6$-$C_{10}$ Aryl z.B. Phenyl, α-Naphthyl, oder β-Naphthyl, insbesondere Phenyl.

Sind $R_{23}$ und $R_{25}$ $C_7$-$C_{14}$ Aralkyl, so handelt es sich z.B. um Benzyl, α-Phenyläthyl oder 2-Phenyläthyl, insbesondere um Benzyl.

$R_{23}$ ist als $C_7$-$C_{14}$ Alkylphenyl z.B. 4-t-Butylphenyl oder 4-Methylphenyl.

$R_{23}$ ist zudem vorzugsweise auch eine Gruppe der Formel VI.

$R_{24}$ ist als $C_7$-$C_{14}$ Aralkyl beispielsweise Benzyl, α-Phenyläthyl oder 2-Phenylpropyl, insbesondere Benzyl. Der Arylrest ist vorzugsweise mit einer Gruppe der Formel Vc substituiert.

$R_{24}$ und $R_{26}$ sind als aliphatischer $C_1$-$C_{18}$ Acylrest beispielsweise Formyl, Acetyl, Propionyl, Butyryl, Octanoyl, Dodecanoyl, Stearoyl oder Acryloyl. $R_{24}$ und $R_{26}$ sind als aromatischer $C_7$ Acylrest Benzoyl und als araliphatischer $C_8$-$C_9$-Acylrest, Cinnamoyl, Phenylacetyl oder Phenylpropionyl. Der aromatische Teil ist gegebenenfalls mit Chlor, $C_1$-$C_4$ Alkyl, wie Methyl, Aethyl, n-Propyl oder t-Butyl oder mit $C_1$-$C_8$ Alkoxy, wie Methoxy, Aethoxy, Butoxy oder Octoxy und/oder Hydroxy substituiert. Substituierte aromatische Acylgruppen sind z.B. Chlorobenzoyl, Toluoyl, Isopropylbenzoyl, 2,4-Dichlorobenzoyl, 4-Methoxybenzoyl, 3-Butoxybenzoyl, 2-Hydroxybenzoyl oder 3,5-Di-t.-butyl-4-hydroxy-benzoyl. Eine araliphatische substituierte Acylgruppe ist beispielsweise β-(3,5-Di-t.-butyl-4-hydroxyphenyl)-propionyl. Sind $R_{24}$ und $R_{26}$ alicyclische $C_6$-$C_9$ Acylgruppen, so kann es sich um Cyclohexylcarbonyl oder 2,4-Dimethylcyclohexylcarbonyl handeln.

Eine bevorzugte Bedeutung von $R_{25}$ ist auch eine Gruppe $-CH_2-CH(R_5)-OR_{26}$.

$R_{28}$ kann als $C_1$-$C_{12}$ Alkylen beispielsweise Methylen, Dimethylen, Trimethylen, Tetramethylen, Hexamethylen, Octamethylen, Decamethylen oder Dodecamethylen sein.

$R_{28}$ bedeutet als $C_6$-$C_{10}$ Arylen beispielsweise Phenylen oder Naphthylen.

Obwohl auch Verbindungen der Formel I, welche bis zu 4 Gruppen der Formel Va, Vb oder VI im Liganden L enthalten durchaus technisch anwendbar sind, werden solche mit 1 oder 2 der genannten Gruppen aus praktischen Gründen bevorzugt. Weitere

Stickstoff-Heterocyclen können jedoch im Aminliganden A auftreten.

Unter den im Molekül auftretenden Gruppen Va, Vb und VI sind vor allem jene der Formel Va und VI hervorzuheben.

$X_1$ hat in der Formel Va die bevorzugte Bedeutung $-\dot{N}R_{25}$ und $X_2$ in der Formel Vc insbesondere -O-.

B ist als einfach geladenes Anion beispielsweise ein solches einer aliphatischen oder aromatischen Carbonsäure, eines Phosphonsäure-Halbesters, einer Phosphinsäure, einer Phosphinigsäure oder eines Enols der Formel IX

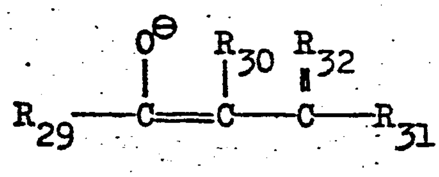

(IX) ,

worin

| | |
|---|---|
| $R_{29}$ | Alkyl, Alkenyl, Cycloalkyl, Aralkyl oder Aryl ist, und |
| $R_{30}$ | Wasserstoff, Alkyl, Alkenyl, Cycloalkyl, Aralkyl, Aryl oder Alkoxycarbonyl ist, oder |
| $R_{29}$ und $R_{30}$ | zusammen gegebenenfalls substituiertes 1,4-Butadi-1,3-enylen oder 1,4-Butylen sind, und |
| $R_{31}$ | gegebenenfalls substituiertes Alkyl, Alkenyl, Cycloalkyl, Aralkyl, Aryl, Alkoxy oder gegebenenfalls substituiertes Amino bedeutet, und |
| $R_{32}$ | Oxo oder gegebenenfalls substituiertes Imino ist. |

Ein einfach geladenes Anion B einer aliphatischen Carbonsäure ist insbesondere ein Anion einer Carbonsäure $R_{33}$-COOH, worin $R_{33}$ ein gegebenenfalls substituierter aliphatischer Kohlenwasserstoffrest ist, welcher insbesondere Alkyl mit 1-25, vor allem 1-12 C-Atomen, Cycloalkyl mit 5-12, insbesondere 5-6 C-Atomen, oder Alkenyl mit 2-25, insbesondere 2-12 C-Atomen und vor allem Vinyl darstellt, die gegebenenfalls durch Halogen, insbesondere Chlor, Hydroxy, Alkyl mit 1-6 C-Atomen oder Alkoxy mit 1-6 C-Atomen substituiert sind.

Beispiele für $R_{33}$ sind Methyl, Aethyl, n-Hexyl, n-Undecyl, 1-Aethyl-n-pentyl, Cyclopentyl, Cyclohexyl, Vinyl, Allyl oder Crotonyl, oder 4-Chlor-butyl.

Ein einfach geladenes Anion B einer aliphatischen Carbonsäure ist auch ein solches einer aliphatischen α- oder β-Aminocarbonsäure, insbesondere einer solchen mit 2-20, vor allem 2-12 C-Atomen, die an der Aminogruppe unsubstituiert ist, vorzugsweise aber an der Aminogruppe mono- oder dialkyliert ist, wie durch verzweigtes oder insbesondere geradkettiges Alkyl mit 1-12, vor allem 1-8 C-Atomen. Beispiele für α-Aminocarbonsäuren sind Glycin, α-Alanin, Valin und Isoleucin, die an der Aminogruppe insbesondere durch n-Alkyl mit 1-8 C-Atomen mono- oder disubstituiert sind, wie Di-n-octyl-glycin. Ein Beispiel für β-Aminocarbonsäuren ist β-Alanin, das an der Aminogruppe insbesondere durch n-Alkyl mit 1-8 C-Atomen mono- oder disubstituiert ist, wie Di-n-propyl-β-alanin.

Ein einfach geladenes Anion B einer aromatischen Carbonsäure ist insbesondere ein Anion einer Carbonsäure $R_{34}$-COOH, worin $R_{34}$ ein gegebenenfalls substituierter aromatischer Kohlenwasserstoffrest ist, welcher insbesondere Aryl mit 6-10 C-Atomen oder Aralkyl mit 7-14 C-Atomen darstellt. Dabei kann es sich vor allem um Phenyl, Benzyl oder Phenyläthyl handeln, welche im Arylteil gegebenenfalls substituiert sein können. Substituenten im Arylteil sind insbesondere Hydroxy, Cyclohexyl,

Phenyl, Benzyl α,α-Dimethylbenzyl und insbesondere $C_1$-$C_{12}$ Alkyl, wie Methyl, Aethyl, Iso-propyl, t.-Butyl, t-Octyl oder n-Dodecyl, insbesondere t-Butyl. Es kann sich bei $R_{34}$ daher beispielsweise um 3,5-Di-t.-butyl-4-hydroxyphenyl-äthyl, 3,5-Di-t.-butyl-4-hydroxybenzyl oder 3,5-Di-t.-butyl-4-hydroxybenzyl oder 3,5-Di-t.-butyl-4-hydroxyphenyl handeln.

Ein einfach geladenes Anion B eines Phosphonsäure-Halbesters oder einer Phosphinsäure ist insbesondere ein Anion eines Phosphonsäure-Halbesters oder einer Phosphinsäure der Formeln X, XI, XII

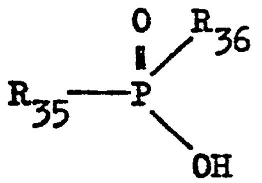 (X) ,

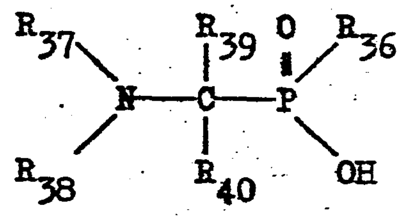 (XI)

oder 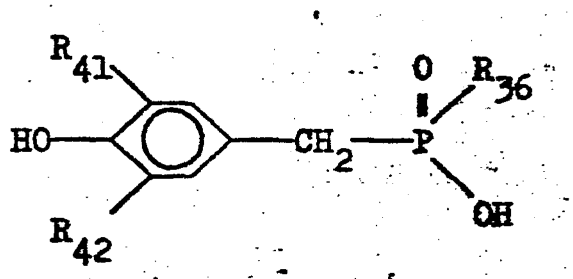 (XII) ,

worin

$R_{35}$ $C_1$-$C_{18}$ Alkyl bedeutet, und

$R_{36}$ $C_1$-$C_8$ Alkyl, Cyclohexyl, gegebenenfalls durch Chlor, $C_1$-$C_4$ Alkyl oder Methoxy monosubstituiertes $C_7$-$C_{11}$ Aralkyl oder Phenyl, $C_1$-$C_4$ Alkoxy, Benzoxy, Phenoxy oder Tolyloxy ist, und

$R_{37}$und$R_{38}$ unabhängig voneinander Wasserstoff, $C_1$-$C_8$ Alkyl, Cyclohexyl, gegebenenfalls mit Chlor, $C_1$-$C_4$ Alkyl und/oder $C_1$-$C_4$ Alkoxy mono- oder disubstituiertes $C_7$-$C_{11}$ Aralkyl oder Phenyl, oder

$R_{37}$und$R_{38}$ zusammen mit dem N-Atom, an das sie gebunden sind, einen Pyrrolidin-, Piperidin-oder Morpholinring bilden, und

$R_{39}$ Wasserstoff, $C_1$-$C_6$ Alkyl, Phenyl oder 4-Methoxyphenyl ist, und

$R_{40}$ Wasserstoff oder Methyl ist, und

$R_{41}$ und $R_{42}$ unabhängig voneinander $C_1$-$C_{12}$ Alkyl bedeuten.

$R_{35}$ ist als $C_1$-$C_{18}$ Alkyl z.B. Methyl, Aethyl, Propyl, n-Butyl, n-Hexyl, n-Octyl, n-Dodecyl, n-Octadecyl, insbesondere $C_1$-$C_4$ Alkyl.

$R_{36}$, $R_{37}$ und $R_{38}$ sind als $C_1$-$C_8$ Alkyl z.B. lineare oder verzweigte Alkylgruppen wie Methyl, Aethyl, Propyl, Isopropyl, n-Butyl, n-Hexyl, n-Octyl oder 2-Aethylhexyl, bevorzugt sind $C_1$-$C_4$ Alkylgruppen.

$R_{36}$, $R_{37}$ und $R_{38}$ sind als $C_7$-$C_{11}$ Aralkyl, welches gegebenenfalls substituiert sein kann, z.B. Benzyl, 4-Methylbenzyl, 4-Isopropylbenzyl, 3-Chlorbenzyl, 4-Methoxybenzyl oder Phenyläthyl, insbesondere Benzyl.

$R_{36}$, $R_{37}$ und $R_{38}$ sind als Phenyl, welches gegebenenfalls substituiert sein kann, beispielsweise Phenyl, 4-Methylphenyl, 3-Chlorphenyl, 4-Methoxyphenyl oder 4-Butoxyphenyl, insbesondere Phenyl.

$R_{36}$ ist als $C_1$-$C_4$ Alkoxy z.B. Methoxy, Aethoxy, Propoxy oder Butoxy.

$R_{37}$ und $R_{38}$ können auch zusammen mit dem Stickstoffatom, an das sie gebunden sind einen Pyrrolidin-, Piperidin- oder Morpholinring, insbesondere einen Piperidin- oder Morpholinring bilden.

$R_{39}$ ist als $C_1$-$C_6$ Alkyl beispielsweise Methyl, Aethyl, Propyl, n-Butyl, oder n-Hexyl, insbesondere Methyl.

Eine weitere bevorzugte Bedeutung von $R_{39}$ ist Wasserstoff.

$R_{41}$ und $R_{42}$ sind als $C_1$-$C_{12}$ Alkyl unabhängig voneinander z.B. Methyl, Aethyl, Isopropyl, t-Butyl, Amyl, Hexyl, t-Octyl oder n-Dodecyl. Bevorzugt sind Alkylgruppen mit 1-4 C-Atomen, insbesondere t-Butyl.

Ein einfach geladenes Anion B einer Phosphinigsäure ist insbesondere ein Anion einer Phosphinigsäure der Formel

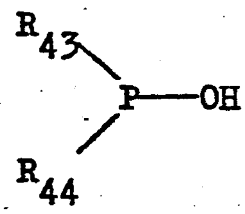

(XIII) ,

worin $R_{43}$ und $R_{44}$ unabhängig voneinander Alkyl, oder gegebenenfalls mit Chlor, Alkyl und/oder Alkoxy substituiertes Phenyl, Benzyl oder Cyclohexyl bedeuten.

Bedeuten $R_{43}$ und $R_{44}$ unabhängig voneinander Alkyl, so handelt es sich insbesondere um Alkyl mit 1-18 C-Atomen, wie Methyl, Aethyl, n-Butyl, n-Hexyl, n-Octyl, n-Dodecyl oder n-Octadecyl.

Bedeuten $R_{43}$ und $R_{44}$ unabhängig voneinander Phenyl, Benzyl oder Cyclohexyl, so können diese gegebenenfalls mit Chlor, $C_1$-$C_4$ Alkyl und/oder $C_1$-$C_4$ Alkoxy mono- oder disubstituiert sein. Es kann sich dabei um 4-Methoxyphenyl, 3-Chlorbenzyl, 4-Butylphenyl, 4-Methylcyclohexyl oder 3-Chlor-4-methylphenyl handeln.

Ist B als einfach geladenes Anion ein Enol der Formel IX, so sind $R_{29}$, $R_{30}$ und $R_{31}$ als Alkyl insbesondere Alkyl mit 1-12 C-Atomen, vor allem mit 1-6 C-Atomen, wie Methyl, Aethyl, n-Propyl, n-Butyl oder n-Hexyl.

$R_{29}$, $R_{30}$ und $R_{31}$ sind als Alkenyl, insbesondere Alkenyl mit 2-12 C-Atomen, vor allem 2-6 C-Atomen wie Allyl, Methallyl oder n-Hex-3-enyl.

$R_{29}$, $R_{30}$ und $R_{31}$ sind als Cycloalkyl insbesondere solche mit 5-12, vor allem 5-6 C-Atomen, wie Cyclohexyl.

$R_{29}$, $R_{30}$ und $R_{31}$ sind als Aralkyl, insbesondere solche mit 7-12 C-Atomen, wie Benzyl oder Phenyläthyl.

$R_{29}$, $R_{30}$ und $R_{31}$ sind als Aryl insbesondere solche mit 6-10 C-Atomen, wie Phenyl, Naphthyl oder Phenyl substituiert mit Alkyl mit 1-12, insbesondere 1-4 C-Atomen wie Methyl, Aethyl oder Butyl; mit Alkoxy mit 1-12, insbesondere 1-4 C-Atomen wie Methoxy oder Butoxy oder mit Halogen wie Chlor.

$R_{30}$ ist als Alkoxycarbonyl insbesondere solches mit 2-12, vor allem 2-6 C-Atomen, wie Methoxycarbonyl oder Aethoxycarbonyl.

Gegebenenfalls substituiertes 1,4-Butadi-1,3-enylen als $R_{29}$ und $R_{30}$ ist insbesondere unsubstituiertes oder solches, das als Substituent z.B. $C_1$-$C_{12}$ Alkyl, insbesondere $C_1$-$C_6$ Alkyl wie Methyl; $C_1$-$C_{12}$ Alkoxy, insbesondere $C_1$-$C_4$ Alkoxy, wie n-Butoxy;oder Halogen, wie Chlor trägt.

$R_{31}$ ist als substituiertes Alkyl insbesondere solches mit 1-12, vor allem 1-6 C-Atomen, das als Substituenten Halogen, wie Chlor trägt.

Bedeutet $R_{31}$ Alkoxy, so ist es insbesondere solches mit 1-12, vor allem 1-6 C-Atomen, wie Methoxy oder Aethoxy.

$R_{31}$ kann eine Aminogruppe bedeuten; ist diese substituiert, so handelt es sich insbesondere um Alkylamino mit 1-12 C-Atomen, wie Methylamino, Dialkylamino mit 2-24 C-Atomen, wie Dimethylamino, und besonders Anilino, das am Phenylring unsubstituiert ist oder als Substituenten in p-, m- oder insbesondere o-Stellung Alkyl mit 1-6 C-Atomen, wie Methyl, oder insbesondere Alkoxy mit 1-6 C-Atomen, wie Methoxy trägt.

Bevorzugte einfach geladene Anionen B sind vor allem solche einer aliphatischen oder aromatischen Carbonsäure, einer Phosphonsäure oder eines Enols der Formel IX.

s hat die Bedeutung 0 bis 2, und ist bevorzugt 0.

Bevorzugt sind Metallkomplexe der Formel XIV

$$\left[M^{q\oplus}\right] \left[L_1^{r\ominus}\right]_{q/r} \cdot mA \qquad \text{(XIV)},$$

worin

M $Mg^{2+}$, $VO^{2+}$, $Ca^{2+}$, $Mn^{2+}$, $Zn^{2+}$, $Co^{2+}$, $Ni^{2+}$ oder $Al^{3+}$ ist, und

q 2 oder 3 ist, und

$L_1$ eine t-zähnige Gruppe der Formel IVa, welche mindestens mit einer Gruppe der Formeln Va, Vb oder VI substituiert ist, bedeutet, und

0, 1 oder 2 ist, und

3 ist, und

$R_{15}$ und $R_{16}$ unabhängig voneinander Wasserstoff, $C_1$-$C_{18}$ Alkyl,

welches gegebenenfalls mit einer Gruppe der Formeln Va, Vb oder Vc substituiert sein kann; oder eine Gruppe Vc oder eine p-ständige Gruppe der Formel VI, Halogen, oder einen Rest $-OR_{24}$ bedeuten oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl, $C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-NR_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cyclo-

alkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

r 1 oder 2 ist, und

m eine Zahl von 0 bis 3 ist, wobei die Summe $(3 \cdot q/r)+$ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aryl, $C_7$-$C_{20}$ Aralkyl, eine gegebenenfalls substituierte Aminoalkylgruppe oder eine Piperidinylgruppe ist, und

$R_{13}$ Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden und

BAD ORIGINAL

$R_{14}$ Wasserstoff oder gegebenenfalls durch -OH substituiertes $C_1$-$C_{18}$ Alkyl bedeutet.

Unter den Verbindungen der Formel XIV sind insbesondere solche zu erwähnen, in welchen

M $Ca^{2+}$, $Mn^{2+}$, $Zn^{2+}$, $Co^{2+}$, $Ni^{2+}$ oder $Al^{3+}$ ist und

q 2 oder 3 ist, und

u 0 ist, und

$L_1$ und t oben angegebene Bedeutung haben, wobei

$R_{15}$ und $R_{16}$ unabhängig voneinander Wasserstoff, $C_1$-$C_8$ Alkyl, welches gegebenenfalls mit einer Gruppe der Formeln Va, Vb oder Vc substituiert sein kann; oder eine Gruppe Vc oder einen Rest $-OR_{24}$ bedeutet, wobei

$R_5$ Wasserstoff oder Methyl ist, und

Y Wasserstoff, $C_1$-$C_4$ Alkyl, $C_3$-$C_6$ Alkenyl, Propargyl, $C_2$-$C_5$ Alkoxyalkyl, Benzyl, Acetyl oder eine der Gruppen $-CH_2COOR_6$, $-CH_2CH(R_7)-OR_8$, $-COOR_9$ oder $-CONHR_9$ ist, worin

$R_6$ $C_1$-$C_4$ Alkyl ist, und

$R_7$ Wasserstoff oder Methyl bedeutet, und

$R_8$ Wasserstoff bedeutet, und

$R_9$ $C_1$-$C_4$ Alkyl ist, und

$X_1$ und $X_2$ unabhängig voneinander -O- oder $-\dot{N}-R_{25}$ sind, und

$R_{21}$ Wasserstoff oder $C_1$-$C_4$ Alkyl, und

$R_{22}$ und Z oben angegebene Bedeutung haben, und

$R_{23}$ $C_1$-$C_{18}$ Alkyl, Allyl, Propargyl, Cyclohexyl, Phenyl, 4-t-Butylphenyl oder eine Gruppe der Formel VI, worin Y und $R_{21}$ die angegebene Bedeutung hat, und

$R_{24}$ Wasserstoff, gegebenenfalls mit einer Gruppe der Formel Vc, worin $X_2$ und $R_{23}$ die angegebene Bedeutung haben, substituiertes $C_1$-$C_{12}$ Alkyl, $C_2$-$C_6$ Alkenyl, Cyclohexyl, Phenyl oder Benzyl bedeutet, und

$R_{25}$ Wasserstoff, $C_1$-$C_{12}$ Alkyl, das gegebenenfalls mit einer Gruppe der Formel Vc, worin $X_2$ und $R_{23}$ die angegebene Bedeutung haben, substituiert sein kann, Allyl, Propargyl, Cyclohexyl, Phenyl, Benzyl oder eine Gruppe $-CH_2-CH(R_5)-OH$, worin $R_5$ die angegebene Bedeutung hat, und

r und m, sowie die Summe $(3 \cdot q/r) + m$ die oben angegebene Bedeutung haben, und

A $H_2O$ oder ein Amin der Formel III ist, worin

$R_{12}$ und $R_{13}$ unabhängig voneinander $C_1$-$C_8$ Alkyl, $C_1$-$C_6$ Hydroxyalkyl oder eine substituierte Aminoalkylgruppe der Formel IId

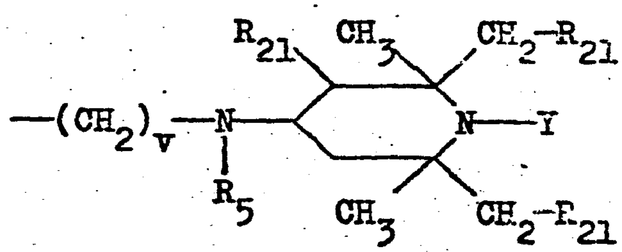

(IId)

bedeutet, worin

v 1 bis 8 ist, und Y, $R_5$ und $R_{21}$ die angegebene Bedeutung hat, oder

$R_{12}$ und $R_{13}$ zusammen eine Piperidingruppe bilden, und

$R_{14}$ Wasserstoff oder $C_1$-$C_8$ Alkyl oder $C_1$-$C_6$ Hydroxyalkyl ist.

Besonders bevorzugte Verbindungen der Formel XIV sind solche, in welchen

M $Ca^{2+}$, $Zn^{2+}$ oder $Ni^{2+}$ ist, und

q 2 ist, und

u 0 ist, und

$L_1$ eine t-zähnige Gruppe der Formel IVa, welche mindestens mit einer Gruppe der Formeln Va oder VI substituiert ist, bedeutet, und

BAD ORIGINAL

t 2 ist, wobei

$R_{15}$ $-OR_{24}$, mit einer Gruppe Va oder Vc substituiertes $C_1$-$C_4$ Alkyl oder eine Gruppe Vc bedeutet, und

$R_{16}$ Wasserstoff oder $C_1$-$C_8$ Alkyl bedeutet, und

$R_5$ Wasserstoff oder Methyl ist, und

Y Wasserstoff, Methyl oder Acetyl ist, und

$X_1$ $-\overset{|}{N}R_{25}$ ist, und

$X_2$ -O- ist, und

$R_{21}$ Wasserstoff oder Methyl ist, und

$R_{23}$ eine Gruppe der Formel VI ist und

$R_{24}$ mit einer Gruppe der Formel Vc, worin $X_2$ und $R_{23}$ die angegebene Bedeutung haben substituiertes $C_1$-$C_4$ Alkyl, bedeutet, und

$R_{25}$ Wasserstoff, $C_1$-$C_{12}$ Alkyl, Cyclohexyl, Benzyl, Phenyl oder eine Gruppe der Formel $-CH_2-CH(R_5)-OH$ ist, und

r 1 ist, und

m 0 bedeutet.

Von ganz besonderem Interesse sind Verbindungen der Formel XIV, worin $R_{15}$ eine o-ständige, mit einer Gruppe der Formel Va substituierte $C_1$-$C_4$ Alkylgruppe bedeutet und $R_{16}$ eine p-ständige 1,1,3,3-Tetramethylbutyl-Gruppe ist.

Beispiele für Verbindungen der Formel XIV sind:

Der $Ni^{2+}$-1:2-Komplex des Di-{2-Hydroxy-5-t-octyl-3-[N-methyl-N-(2,2,6,6-tetramethyl-piperidin-4-yl)-amino-methyl]-phenyl}-sulfids.

Der $Ni^{2+}$ oder $Ca^{2+}$-1:2-Komplex des Di-{2-Hydroxy-5-t-octyl-3-[1,2,2,6,6-pentamethyl-piperidin-4-oxycarbonyl-methyl] phenyl}-sulfids.

Der $Zn^{2+}$-1:1-Komplex des Di-{2-Hydroxy-5-[1,2,2,6,6-pentamethyl-piperidin-4-yl]-phenyl}-sulfids · (4-Amino-1,2,2,6,6-pentamethylpiperidin)$_3$.

Der $Ni^{2+}$-1:2-Komplex des Di-{2-Hydroxy-5-[2,2,6,6-tetramethyl-piperidin-4-yl]-phenyl}-sulfids.

Der $Ca^{2+}$ oder $Ni^{2+}$-1:1-Komplex des Di-{2-Hydroxy-5-t-octyl-3-[2,2,6,6-tetramethyl-piperidin-4-oxy-carbonyl-methyl]-phenyl}-sulfids · (4-Amino-2,2,6,6-tetramethyl-piperidin)$_3$.

Der $Ni^{2+}$-1:1-Komplex des Di-{2-Hydroxy-5-t-octyl-3-[N-(2,3,6-trimethyl-2,6-diäthyl-piperidin-4-yl)-amino]-phenyl}-sulfids · (Triäthanolamin)$_3$.

Ferner sind Metallkomplexe der Formel XV bevorzugt

$$\left[M^{q\oplus}\right] \left[L_2^{r\ominus}\right]_{q/r} \cdot mA \qquad (XV),$$

worin

M $Mg^{2+}$, $VO^{2+}$, $Ca^{2+}$, $Mn^{2+}$, $Zn^{2+}$, $Co^{2+}$, $Ni^{2+}$ oder $Al^{3+}$ ist, und

q 2 oder 3 ist, und

$L_2$ eine t-zähnige Gruppe der Formel IVb, welche mindestens mit einer Gruppe der Formeln Va, Vb oder VI substituiert ist, bedeutet, und

t 2 ist, und

$R_{15}$ und $R_{16}$ unabhängig voneinander Wasserstoff, $C_1$-$C_{18}$ Alkyl,

welches gegebenenfalls mit einer Gruppe der Formeln Va, Vb oder Vc substituiert sein kann oder eine Gruppe Vc oder eine p-ständige Gruppe der Formel VI, Halogen oder einen Rest der Formel $-OR_{24}$ bedeuten oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl, $C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-\dot{N}R_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{17}$ Wasserstoff, $C_1$-$C_{18}$ Alkyl, Halogen, eine Gruppe $-OR_{24}$ oder eine Gruppe der Formel Vc, worin $R_{23}$, $R_{24}$ und $X_2$ die angegebene Bedeutung haben, bedeutet, und

r 1 ist, und

m eine Zahl von 0 bis 2 ist, wobei die Summe $(2\cdot q)+m$ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aryl, $C_7$-$C_{20}$ Aralkyl, eine gegebenenfalls substituierte Aminoalkylgruppe oder eine Piperidinylgruppe ist, und

| | |
|---|---|
| $R_{13}$ | Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder |
| $R_{12}$ und $R_{13}$ | zusammen mit dem N-Atom einen durch Alkylgruppen substituierten oder unsubstituierten Pyrrolidin-, Piperidin- oder Morpholinring bilden, und |
| $R_{14}$ | Wasserstoff oder gegebenenfalls mit -OH substituiertes $C_1$-$C_{18}$ Alkyl bedeutet. |

Unter den Verbindungen der Formel XV sind insbesondere solche zu erwähnen, in welchen

| | |
|---|---|
| M | $Ca^{2+}$, $Mn^{2+}$, $Zn^{2+}$, $Co^{2+}$, $Ni^{2+}$ oder $Al^{3+}$ ist, und |
| q | 2 oder 3 ist, und |
| $L_2$ und t | die oben angegebene Bedeutung haben, wobei |
| $R_{15}$ und $R_{16}$ | unabhängig voneinander Wasserstoff, $C_1$-$C_8$ Alkyl, welches gegebenenfalls mit einer Gruppe der Formeln Va, Vb oder Vc substituiert sein kann; oder eine Gruppe Vc, oder einen Rest $-OR_{24}$ bedeutet, wobei |
| $R_5$ | Wasserstoff oder Methyl ist, und |
| Y | Wasserstoff, $C_1$-$C_4$ Alkyl, $C_3$-$C_6$ Alkenyl, Propargyl, $C_2$-$C_5$ Alkoxyalkyl, Benzyl, Acetyl oder eine der Gruppen $-CH_2COOR_6$, $-CH_2CH(R_7)-OR_8$, $-COOR_9$ oder $-CONHR_9$ ist, worin |
| $R_6$ | $C_1$-$C_4$ Alkyl ist, und |
| $R_7$ | Wasserstoff oder Methyl bedeutet, und |
| $R_8$ | Wasserstoff bedeutet, und |
| $R_9$ | $C_1$-$C_4$ Alkyl ist, und |
| $X_1$ und $X_2$ | unabhängig voneinander -O- oder $-\dot{N}-R_{25}$ sind, und |
| $R_{21}$ | Wasserstoff oder $C_1$-$C_4$ Alkyl, und |

$R_{22}$ und Z oben angegebene Bedeutung haben, und

$R_{23}$ $C_1$-$C_{18}$ Alkyl, Allyl, Propargyl, Cyclohexyl, Phenyl, 4-t-Butylphenyl oder eine Gruppe der Formel VI, worin Y und $R_{21}$ die angegebene Bedeutung hat, und

$R_{24}$ Wasserstoff, gegebenenfalls mit einer Gruppe der Formel Vc, worin $X_2$ und $R_{23}$ die angegebene Bedeutung haben, substituiertes $C_1$-$C_{12}$ Alkyl, $C_2$-$C_6$ Alkenyl, Cyclohexyl, Phenyl oder Benzyl bedeutet, und

$R_{25}$ Wasserstoff, $C_1$-$C_{12}$ Alkyl, das gegebenenfalls mit einer Gruppe der Formel Vc, worin $X_2$ und $R_{23}$ die angegebene Bedeutung haben, substituiert sein kann, Allyl, Propargyl, Cyclohexyl, Phenyl, Benzyl oder eine Gruppe $-CH_2-CH(R_5)-OH$, worin $R_5$ die angegebene Bedeutung hat, und

$R_{17}$ Wasserstoff, $C_1$-$C_{12}$ Alkyl, einen Rest $-OR_{24}$ oder eine Gruppe der Formel Vc, worin $R_{23}$, $R_{24}$ und $X_2$ die angegebene Bedeutung haben, und

r und m, sowie die Summe $(2 \cdot q)+m$ die oben angegebene Bedeutung haben, und

A $H_2O$ oder ein Amin der Formel III ist, worin

$R_{12}$ und $R_{13}$ unabhängig voneinander $C_1$-$C_8$ Alkyl, $C_1$-$C_6$ Hydroxyalkyl oder eine substituierte Aminoalkylgruppe der Formel IId

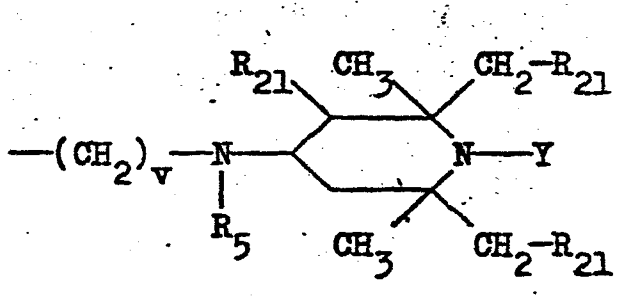

(IId)

bedeutet, worin

v 1 bis 8 ist, und Y, $R_5$ und $R_{21}$ die angegebene Bedeutung haben, oder

| | |
|---|---|
| $R_{12}$ und $R_{13}$ | zusammen eine Piperidingruppe bilden, und |
| $R_{14}$ | Wasserstoff oder $C_1$-$C_8$ Alkyl oder $C_1$-$C_6$ Hydroxyalkyl ist. |

Besonders bevorzugte Verbindungen der Formel XV sind solche, in welchen

| | |
|---|---|
| M | $Ca^{2+}$, $Zn^{2+}$ oder $Ni^{2+}$ ist, und |
| q | 2 ist, und |
| $L_2$ | eine t-zähnige Gruppe der Formel IVb, welche mindestens mit einer Gruppe der Formeln Va oder VI substituiert ist, bedeutet, und |
| t | 2 ist, wobei |
| $R_{15}$ | Wasserstoff, $C_1$-$C_5$ Alkyl, einen Rest $-OR_{24}$, mit einer Gruppe Va oder Vc substituiertes $C_1$-$C_4$ Alkyl oder eine Gruppe Vc bedeutet, und |
| $R_{16}$ | Wasserstoff oder $C_1$-$C_8$ Alkyl bedeutet, und |
| $R_{17}$ | Wasserstoff ist, und |
| $R_5$ | Wasserstoff oder Methyl ist, und |
| Y | Wasserstoff, Methyl oder Acetyl ist, und |
| $X_1$ | $-\dot{N}-R_{25}$ bedeutet, und |
| $X_2$ | -O- ist, und |
| $R_{21}$ | Wasserstoff oder Methyl ist, und |
| $R_{23}$ | eine Gruppe der Formel VI bedeutet, und |
| $R_{24}$ | mit einer Gruppe der Formel Vc, worin $X_2$ und $R_{23}$ die angegebene Bedeutung haben, substituiertes $C_1$-$C_4$ Alkyl bedeutet, und |
| $R_{25}$ | Wasserstoff, $C_1$-$C_{12}$ Alkyl, Cyclohexyl, Benzyl, $-CH_2-CH(R_5)-OH$ ist, und |

r, m, die Summe $(2 \cdot q)+m$, A, $R_{12}$, $R_{13}$ und $R_{14}$ die oben angegebene Bedeutung haben.

Von ganz besonderem Interesse sind Verbindungen der Formel XV, worin $R_{15}$ eine zur Hydroxygruppe o-ständige, mit einer Gruppe der Formel Va substituierte $C_1$-$C_4$ Alkylgruppe oder insbesondere auch eine zur Hydroxygruppe m-ständige Gruppe der Formel $-OR_{24}$, worin $R_{24}$ eine mit einer Gruppe der Formel Vc substituierte $C_1$-$C_4$ Alkylgruppe ist, und $R_{16}$ und $R_{17}$ Wasserstoff sind.

Beispiele von Verbindungen der Formel XV sind:

Der $Ni^{2+}$-1:2-Komplex des 2-(2'-Hydroxy-5'-t-butylphenyl)-5-(N-benzyl-2,2,6,6-tetramethyl-piperidin-4-oxy-carbonyl)-benztriazols · (Triäthanolamin)$_2$.

Der $Ca^{2+}$-1:2-Komplex des 2-{2'-Hydroxy-5'-t-butyl-4'-[2*-di-(1,2,2,6,6-pentamethyl-piperidin-4-oxy-carbonyl)-hexyl]-phenyl}-benztriazols · (4-Amino-2,2,6,6-tetramethyl-piperidins)$_2$.

Der $Zn^{2+}$-1:2-Komplex des 2-{2'-Hydroxy-5'-sec-butyl)-4'-[2*-di-(1-acetyl-2,2,6,6-tetramethylpiperidin-4-oxy-carbonyl)-hexyl]-phenyl}-4-(1-acetyl-2,2,6,6-tetramethyl-piperidin-4-oxycarbonyl)-benztriazols · (4-Dimethyl-amino-2,2,6,6-tetramethyl-piperidin)$_2$.

Der $Ni^{2+}$-1:2-Komplex des 2-{2'-Hydroxy-3'-sec-butyl)-4'-[1,2,2,6,6-pentamethyl-piperidin-4-oxycarbonyl]-phenyl}-benztriazols · (N,N'-Methyl-N,N'-(1,2,2,6,6-pentamethyl-piperidin-4-yl)-äthylendiamin.

Ferner sind Metallkomplexe der Formel XVI bevorzugt

$$\left[M^{q\oplus}\right] \left[L_z^{r\ominus}\right]_{q/r} \cdot mA \qquad (XVI),$$

worin

M $Mg^{2+}$, $VO^{2+}$, $Ca^{2+}$, $Mn^{2+}$, $Zn^{2+}$, $Co^{2+}$, $Ni^{2+}$ oder $Al^{3+}$ ist, und

q 2 oder 3 ist, und

$L_3$ eine t-zähnige Gruppe der Formel IVc, welche mindestens mit einer Gruppe der Formeln Va, Vb oder VI substituiert ist, bedeutet, und

t 2 oder 4 ist, und

$R_{15}$ und $R_{16}$ unabhängig voneinander Wasserstoff, $C_1$-$C_{18}$ Alkyl, welches gegebenenfalls mit einer Gruppe der Formeln Va, Vb oder Vc substituiert sein kann; oder eine Gruppe Vc, eine p-ständige Gruppe der Formel VI, Halogen oder einen Rest $-OR_{24}$ bedeuten oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl, $C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe. eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-NR_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_6$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{18}$ eine Gruppe $-N(R_{25})R_{27}$ ist, und

$R_{19}$ Wasserstoff ist, oder

$R_{18}$ und $R_{19}$ zusammen =O oder $=NR_{27}$ sind, und

$R_{20}$ Wasserstoff, $C_7$-$C_{14}$ Aralkyl, $C_7$-$C_{14}$ Alkylphenyl, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, eine Gruppe der Formel VII, oder falls $R_{18}$ und $R_{19}$ zusammen =O sind eine Gruppe der Formel Va; wobei $X_1$, $R_{21}$ und Y oben definierte Bedeutung haben, und

$R_{28}$ $C_1$-$C_{12}$ Alkylen, Butenylen, $C_6$-$C_{10}$ Arylen oder Diphenylen ist, bedeutet, und

$R_{27}$ Wasserstoff, $C_7$-$C_{14}$ Aralkyl, $C_7$-$C_{14}$ Alkylphenyl, eine Gruppe $-OR_{24}$, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder eine Gruppe der Formel VI, oder eine Gruppe der Formel

$-CH_2-CH(R_5)-OR_{26}$; oder falls $R_{20}$ nicht eine Gruppe der Formel VII ist; $R_{27}$ auch eine Gruppe der Formel VIIIa oder VIIIb bedeutet, und

r 1 oder 2 ist, und

m eine Zahl von 0 bis 2 ist, wobei die Summe $(t \cdot q/r)+m$ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aralkyl, $C_7$-$C_{20}$ Aralkyl, eine gegebenenfalls substituierte Aminoalkylgruppe oder eine Piperidinylgruppe ist, und

$R_{13}$ Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden, und

$R_{14}$ Wasserstoff oder gegebenenfalls mit -OH substituiertes $C_1$-$C_{18}$ Alkyl bedeutet.

Unter den Verbindungen der Formel XVI sind vor allem solche zu erwähnen, in welchen

M $Ca^{2+}$, $Mn^{2+}$, $Zn^{2+}$, $Co^{2+}$, $Ni^{2+}$ oder $Al^{3+}$ ist, und

q 2 oder 3 ist, und

t 2 ist

$L_3$ oben angegebene Bedeutung hat, wobei

$R_{15}$ und $R_{16}$ unabhängig voneinander Wasserstoff, $C_1$-$C_8$ Alkyl, welches gegebenenfalls mit einer Gruppe der Formeln Va, Vb oder Vc substituiert sein kann; oder eine Gruppe Vc oder einen Rest $-OR_{24}$ bedeutet, wobei

$R_5$ Wasserstoff oder Methyl ist, und

Y Wasserstoff, $C_1$-$C_4$ Alkyl, $C_3$-$C_6$ Alkenyl, Propargyl, $C_2$-$C_5$ Alkoxyalkyl, Benzyl, Acetyl oder eine der Gruppen $-CH_2COOR_6$, $-CH_2CH(R_7)-OR_8$, $-COOR_9$ oder $-CONHR_9$ ist, worin

$R_6$ $C_1$-$C_4$ Alkyl ist, und

$R_7$ Wasserstoff oder Methyl bedeutet, und

$R_8$ Wasserstoff bedeutet, und

$R_9$ $C_1$-$C_4$ Alkyl ist, und

$X_1$ und $X_2$ unabhängig voneinander -O- oder $-\overset{|}{N}-R_{25}$ sind, und

$R_{21}$ Wasserstoff oder $C_1$-$C_4$ Alkyl, und

$R_{22}$ und Z oben angegebene Bedeutung haben, und

$R_{23}$ $C_1$-$C_{18}$ Alkyl, Allyl, Propargyl, Cyclohexyl, Phenyl, 4-t-Butylphenyl oder eine Gruppe der Formel VI, worin Y und $R_{21}$ die angegebene Bedeutung hat, und

$R_{24}$ Wasserstoff, gegebenenfalls mit einer Gruppe der Formel Vc, worin $X_2$ und $R_{23}$ die angegebene Bedeutung haben, substituiertes $C_1$-$C_{12}$ Alkyl, $C_2$-$C_6$ Alkenyl, Cyclohexyl, Phenyl oder Benzyl bedeutet, und

$R_{25}$ Wasserstoff, $C_1$-$C_{12}$ Alkyl, das gegebenenfalls mit einer Gruppe der Formel Vc, worin $X_2$ und $R_{23}$ die angegebene Bedeutung haben, substituiert sein kann, Allyl, Propargyl, Cyclohexyl, Phenyl, Benzyl oder eine Gruppe $-CH_2-CH(R_5)-OH$, worin $R_5$ die angegebene Bedeutung hat, und

$R_{18}$ eine Gruppe $-N(R_{25})R_{27}$ ist, und

$R_{19}$ Wasserstoff ist, oder

$R_{18}$ und $R_{19}$ zusammen =O oder $=NR_{27}$ sind, und

$R_{20}$ Wasserstoff, oder gegebenenfalls mit einer Gruppe der Formel Vc substituiertes $C_1$-$C_{12}$ Alkyl, $C_2$-$C_6$ Alkenyl, Cyclohexyl oder Phenyl ist, und falls $R_{18}$ $R_{19}$ zusammen =O sind, eine Gruppe der Formel Va ist, und

$R_{27}$ Wasserstoff, oder gegebenenfalls mit einer Gruppe der Formel Vc substituiertes $C_1$-$C_{12}$ Alkyl, $C_2$-$C_6$ Alkenyl, Cyclohexyl oder Phenyl; oder eine Gruppe der Formel VI bedeutet, und

r 1 ist, und

m eine Zahl von 0 bis 2 ist, wobei die Summe $(2\cdot q)+m$ gleich der Koordinationszahl des Metallions M ist, und

A $H_2O$ oder ein Amin der Formel III ist, worin

$R_{12}$ und $R_{13}$ unabhängig voneinander $C_1$-$C_8$ Alkyl, $C_1$-$C_6$ Hydroxyalkyl oder eine substituierte Aminoalkylgruppe der Formel IId

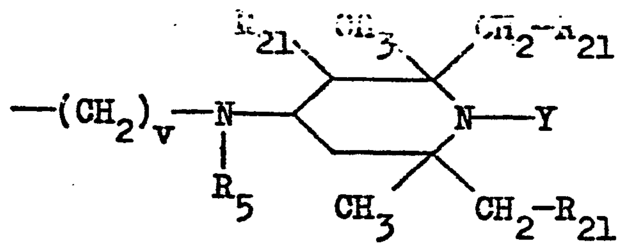

(11a)

bedeutet, worin

v 1 bis 8 ist, und Y, $R_5$ und $R_{21}$ die angegebene Bedeutung hat, oder

$R_{12}$ und $R_{13}$ zusammen eine Piperidingruppe bilden, und

$R_{14}$ Wasserstoff oder $C_1$-$C_8$ Alkyl oder $C_1$-$C_6$ Hydroxyalkyl ist.

Besonders bevorzugte Verbindungen der Formel XVI sind solche, in denen

M $Ca^{2+}$, $Zn^{2+}$ oder $Ni^{2+}$ ist, und

q 2 ist, und

L eine t-zähnige Gruppe der Formel IVc, welche mindestens mit einer Gruppe der Formel VI substituiert ist, bedeutet, und

t 2 ist, wobei

$R_{15}$ und $R_{16}$ Wasserstoff sind, und

$R_{18}$ eine Gruppe $-N(R_{25})R_{27}$ ist, worin

$R_{25}$ Wasserstoff, $C_1$-$C_{12}$ Alkyl oder eine Gruppe $-CH_2-CH(R_5)-OH$, worin $R_5$ Wasserstoff oder Methyl ist, bedeutet, und

$R_{27}$ Wasserstoff, oder mit einer Gruppe der Formel Vc substituiertes $C_1$-$C_4$ Alkyl oder eine Gruppe der Formel VI bedeutet, wobei

$X_2$ -O- ist, und

Y Wasserstoff, Methyl oder Acetyl bedeutet, und

$R_{21}$ Wasserstoff oder Methyl ist, und
$R_{23}$ eine Gruppe der Formel VI ist, und
$R_{19}$ Wasserstoff ist, und
$R_{20}$ Wasserstoff oder $C_1$-$C_4$ Alkyl ist
r,m,A,$R_{12}$,$R_{13}$ und $R_{14}$ oben angegebene Bedeutung haben.

Besonders bevorzugte Verbindungen der Formel XVI sind auch solche, in denen

M $Ca^{2+}$, $Zn^{2+}$ oder $Ni^{2+}$ ist, und
q 2 ist, und
L eine t-zähnige Gruppe der Formel IVc, welche mindestens mit einer Gruppe der Formeln Va oder VI substituiert ist, bedeutet, und
t 2 ist, und
$R_{18}$ und $R_{19}$ zusammen =O sind, und
$R_{15}$ Wasserstoff, $C_1$-$C_4$ Alkyl, welches gegebenenfalls mit einer Gruppe der Formeln Va oder VI substituiert ist oder eine Gruppe Vc oder einen Rest $-OR_{24}$ bedeutet, und
$R_{16}$ Wasserstoff ist, wobei
$X_1$ $-NR_{25}$ bedeutet, und
$R_{21}$ Wasserstoff oder Methyl ist, und
Y Wasserstoff, Methyl oder Acetyl ist, und
$R_{24}$ mit einer Gruppe der Formel Vc substituiertes $C_1$-$C_4$ Alkyl bedeutet, wobei
$R_{23}$ eine Gruppe der Formel VI und
$X_2$ -O- bedeutet, und
$R_{16}$ Wasserstoff ist, und
$R_{20}$ Wasserstoff, $C_1$-$C_5$ Alkyl, mit einer Gruppe der Formel Vc substituiertes $C_1$-$C_4$ Alkyl oder $C_2$ Alkenyl oder eine Gruppe der Formel Va, worin $X_2$, $R_{21}$ und Y die angegebene Bedeutung haben, und

$R_{25}$ Wasserstoff, $C_1$-$C_{12}$ Alkyl oder eine Gruppe $-CH_2-CH(R_5)-OH$, worin $R_5$ Wasserstoff oder Methyl ist, bedeutet, und

r,m,A,$R_{12}$,$R_{13}$ und $R_{14}$ oben angegebene Bedeutung haben.

Besonders bevorzugte Verbindungen der Formel XVI sind auch solche, in denen

M $Ca^{2+}$, $Zn^{2+}$ oder $Ni^{2+}$ ist, und

q 2 ist, und

L eine t-zähnige Gruppe der Formel IVc, welche mindestens mit einer Gruppe der Formel Va oder VI substituiert ist, bedeutet, und

t 2 ist, und

$R_{18}$ und $R_{19}$ zusammen $=NR_{27}$ sind, und

$R_{15}$ Wasserstoff, $C_1$-$C_4$ Alkyl, welches gegebenenfalls mit einer Gruppe der Formeln Va oder VI substituiert ist oder eine Gruppe Vc, oder einen Rest $-OR_{24}$ bedeutet, und

$R_{16}$ Wasserstoff ist, wobei

$X_1$ $-\overset{|}{N}-R_{25}$ bedeutet, und

$R_{21}$ Wasserstoff oder Methyl ist, und

Y Wasserstoff, Methyl oder Acetyl ist, und

$R_{24}$ mit einer Gruppe der Formel Vc substituiertes $C_1$-$C_4$ Alkyl bedeutet, wobei

$R_{23}$ eine Gruppe der Formel VI, und

$X_2$ -O- bedeutet, und

$R_{20}$ Wasserstoff, $C_1$-$C_5$ Alkyl oder mit einer Gruppe der Formel Vc substituiertes $C_1$-$C_4$ Alkyl ist, und

$R_{25}$ Wasserstoff, $C_1$-$C_{12}$ Alkyl oder eine Gruppe $-CH_2-CH(R_5)-OH$, worin $R_5$ Wasserstoff oder Methyl ist, und

$R_{27}$ eine Gruppe der Formel VI bedeutet, und
r, m, A, $R_{12}$, $R_{13}$ und $R_{14}$ oben angegebene Bedeutung haben.

Beispiele für Verbindungen der Formel XVI sind:

Der $Ni^{2+}$-1:2-Komplex des N-Methyl-N-(2-hydroxyphenyl)-N-[1',2',2',6',6'-pentamethyl-piperidin-4'-oxy-carbonylmethyl]-azomethins · (4-Amino-2,2,6,6-tetramethyl-piperidin)$_2$.

Der $Al^{3+}$-1:3-Komplex des N-Phenyl-N-(2-hydroxyphenyl)-N-[2'-(1-benzyl-2,2,6,6-tetramethyl-piperidin-4-oxy-carbonyl)-phenyl]-azomethins.

Der $Ni^{2+}$-1:1-Komplex des Hexamethylen-di-{N-methyl-[2-hydroxy-5-(1',2',2',6',6'-pentamethyl-piperidin-4'-yl)]}-azomethins · (Triäthanolamin)$_2$.

Der $Ca^{2+}$-1:2-Komplex des N-(1,2,2,6,6-pentamethyl-piperidin-4-yl)-N-n-butyl-N-([2'-hydroxy-5'-t-octyl]-benzyl)-amins · (Dibenzylpropylamin)$_2$.

Der $Ni^{2+}$-1:2-Komplex des Hexamethylen-di-{N-[2-hydroxy-4-(1'-hexyl-2',2',6',6'-tetramethyl-piperidin-4'-oxy-carbonyl-methyl-oxy)}-benzyl-amin · (Triäthanolamin)$_2$.

Der $Mg^{2+}$-1:2-Komplex des 2-{1',2',2',6',6'-Pentamethylpiperidin-4-oxycarbonyl}-4-{1',2',2',6',6'-pentamethyl-piperidin-4-oxycarbonyl-methyl-oxy}-phenols · (4-Dimethylamin-2,2,6,6-tetramethyl-piperidin)$_2$.

Der $Ni^{2+}$-1:2-Komplex des 2- Methyl-carbonyl{-3,5-di- 2',2',6',6'-tetramethyl-piperidin-4-oxycarbonyl-methyloxy}-phenols · (Triäthanolamin)$_2$.

Die Herstellung der Verbindungen der Formel I ist bekannt.

So erhält man die Verbindungen der Formel I, worin s und m 0 sind am einfachsten dadurch, dass man ungefähr ein Mol des freien Liganden $[L]H_r$ mit ungefähr $r/_q*$ Molen eines Alkali- oder Erdalkalihydroxids, -alkoholats, -hydrids oder -amids oder auch $r/_{2q}*$ Molen eines Alkali- oder Erdalkalicarbonats in einem inerten, organischen Lösungsmittel umsetzt. Die Reaktion kann bei Raumtemperatur oder zur Beschleunigung der Umsetzung bei erhöhten Temperaturen bis zur Rückflusstemperatur erfolgen. Am einfachsten arbeitet man bei Raumtemperatur. Als Alkalimetalle kommen insbesondere Lithium, Natrium, Kalium oder Rubidium, vor allem Natrium oder Kalium in Frage. Als Erdalkalimetall eignet sich insbesondere Kalzium. q* definiert die Ladung des verwendeten Alkali- oder Erdalkalimetallkations M*. Als inerte, organische Lösungsmittel eignen sich Alkohole, wie Methanol oder Aethanol, Aether, wie Dioxan Tetrahydrofuran oder Diäthyläther; aromatische Kohlenwasserstoffe, wie Benzol, Toluol, Xylol; aliphatische Kohlenwasserstoffe wie Hexan oder Ligroin; oder Amide wie Dimethylformamid oder Hexamethylphosphorsäuretriamid.

Die auf diese Weise erhaltenen Alkali- bzw. Erdalkalisalze $[L]M*_{r/q*}$ können anschliessend auf einfache Weise mit einem Metallsalz $ML*_q$ umgesetzt werden. Es gelangen dabei vor allem $q/_r$ Mole der Verbindung $[L]M*_{r/q*}$ pro Mol $ML*_q$ in einem inerten organischen Lösungsmittel zum Einsatz. Als Lösungsmittel eignen sich die oben beschriebenen. Die Temperatur kann wiederum von Raumtemperatur bis zur Rückflusstemperatur gewählt werden. Als Anion L* eignen sich insbesondere Halogenid vor allem Chlorid, oder Carboxylatanionen, wie Acetat oder Capronat.

Es ist auch möglich,die beiden oben beschriebenen Verfahrensschritte zu vereinen oder Hydrate der beschriebenen Verbindungen einzusetzen.

Wird als $ML^*_q$ z.B. ein Hydroxid oder Carbonat verwendet, so kann auf die Alkali- bzw. Erdalkalimetallsalzbildung sogar verzichtet werden.

Falls s nicht 0 ist, wird grundsätzlich wie beschrieben vorgegangen, wobei $^{(q-s)}/r$ Mole einer Verbindung $[L]M^*_{r/q}$ und s Mole einer Verbindung BM* mit einem Metallsalz der Formel $ML^*_q$ umgesetzt werden. Zum gleichen Ergebnis kommt man durch Ligandenaustauschreaktionen, wobei z.B. $MB_q$ mit $^{(q-s)}/r$ Molen $[L]M^*_{r/q}$ zum Einsatz gelangen. Die Verbindungen B sind bekannt und nach bekannten Methoden herstellbar.

Soll die Verbindung der Formel I ein Amin A enthalten, so wird am einfachsten eine Lösung der Verbindung $[M^{q+}][L^{r-}]_{(q-s)/r} \cdot [B^-]_s$, deren Herstellung oben beschrieben ist, in einem der beschriebenen aprotischen inerten Lösungsmittel in Gegenwart von mindestens m Molen der Verbindung A erhitzt. Die Temperatur kann bis zur Rückflusstemperatur betragen, soll auf jeden Fall aber mindestens 30°C sein. Vorteilhafte Resultate erzielt man durch Kochen unter Rückfluss, wobei ein Wasserabscheider verwendet wird.

Die Amine A sind bekannt, und nach bekannten Methoden herstellbar. Einige solcher Verbindungen sind beispielsweise im US-Pat. 3,901,031 oder falls A Piperidylreste enthält auch in der DT-OS 2,349,962 beschrieben.

Die Bedingung, welche ein chelatbildender Ligand gemäss der

vorliegenden Erfindung erfüllen muss, ist, dass er mindestens eine Gruppe der Formel IIa oder IIb enthält. Wie man zu Verbindungen kommt, welche Gruppen IIa oder IIb enthalten ist in der Literatur beschrieben worden. So sind beispielsweise Derivate des Pyrrolidins in der DT-OS 2,459,331; des Imidazolidins in der DT-OS 2,427,853 oder der DT-OS 2,500,313; des 1,4-Diazacycloheptanons in der DT-OS 2,428,877 oder der DT-OS 2,621,924;des Piperazins in der DT-OS 2,315,042; des 1-Aza-4-thiacyclohexans in der DT-OS 1,351,865; des Dipiperidyls in der DT-OS 2,425,984 und des Piperidins z.B. in den DT-OS 2,258,752, 2,040,975; 2,227,689, den US-Pat. No. 3,639,409, 3,640,928 oder dem GB-Pat.No. 1,262,234 beschrieben.

Wie bereits erwähnt, sind diejenigen Chelatbildner, welche einen oder mehrere Piperidinreste enthalten, von besonderem Interesse. Dies gelangt auch dadurch zum Ausdruck, dass die besonders bevorzugten Verbindungen Gruppen der Formel Va, Vb oder VI enthalten, zu denen man beispielsweise wie folgt gelangt:

Handelt es sich um eine Gruppe der Formel Va oder VI,so kann von den entsprechenden 4-Hydroxypiperidinen (bekannt aus der DT-OS 2,352,658) oder den 4-Aminopiperidinen (bekannt aus dem US-Pat. 3,684,765) ausgegangen werden. Die 4-OH-Verbindungen können allgemein aus den 4-Oxopiperidinen der Formel XVII

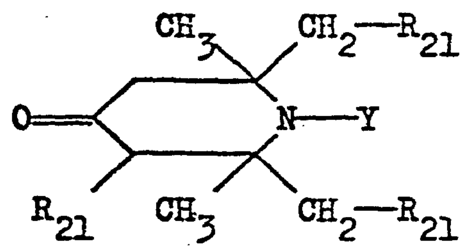

(XVII)

in der $R_{21}$ und Y die oben angegebene Bedeutung haben, durch Reduktion, z.B. katalytische Hydrierung über Raney-Nickel hergestellt werden, während die 4-$NH_2$-Verbindungen z.B. mittels reduktiver Umsetzung mit Ammoniak aus einer Verbindung der Formel XVII erhältlich sind.

Die 4-Oxopiperidine der Formel XVII, worin Y Wasserstoff ist, können nach verschiedenen Verfahren hergestellt werden.

So wird z.B. von W. Traube in Chem. Ber. 41, 777 (1908) die Umsetzung von einem aliphatischen Keton mit Ammoniak beschrieben.

4-Oxopiperidine der Formel XVII, worin Y Wasserstoff bedeutet können auch analog dem in US-Pat. 3,513,170 beschriebenen Verfahren hergestellt werden. Dabei wird ein alkylsubstituiertes Tetrahydropyrimidin in Gegenwart eines sauren Katalysators hydrolytisch umgelagert.

N-H-Verbindungen der Formel XVII, welche in 2- und 6-Stellun verschiedenartige Substituenten besitzen, können durch Umsetzung eines Ketones der Formel $CH_3$-CO-$CH_2R_{21}$ mit Ammoniak hergestellt werden. Das gebildete Pyrimidin wird, wie in Helv. Chim. Acta 30, 114 (1947) beschrieben, zu einem Aminoketon der Formel XVIII hydrolisiert.

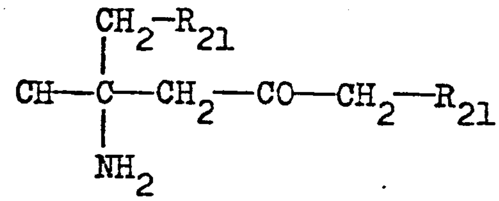

(XVIII)

Die Verbindungen der Formel XVIII werden in einem zweiten Verfahrensschritt mit Ammoniak und einem Keton $CH_3$-CO-$CH_2$-$R_{21}$ umgesetzt, wie es z.B. in Monatsh. Chemie 88, 464 (1957) beschrieben ist. Die Verbindungen der Formel XVII, worin Y Wasserstoff bedeutet, können aus dem so erhaltenen Pyrimidin durch Hydrolyse gewonnen werden.

Verbindungen der Formel XVII, worin Y nicht Wasserstoff bedeutet, oder auch solche, die der protonierten Form der Gruppe der Formel Vb entsprechen, können durch Substitution aus den entsprechenden N-H-Verbindungen hergestellt werden. Es handelt sich dabei um die für sekundäre Amine üblichen Substitutionsreaktionen, obwohl diese, bedingt durch die sterische Hinderung durch die Methylgruppe bzw. die Gruppe -$CH_2$-$R_{21}$ langsamer verlaufen. Die N-H-Verbindungen können z.B. mit Alkyl-, Alkenyl-, Aralkyl- oder Alkoxyalkylhalogeniden, mit Dialkylsulfaten, mit Epichlorhydrinen, mit Estern von Chlor-Carbonsäuren wie Chloressigsäureestern oder Säurechloriden bzw. -anhydriden umgesetzt werden.

Die Gruppe -$CH_2$-CH($R_7$)-$OR_8$ kann durch Umsetzung der N-H-Piperidine mit einem Epoxid der Formel 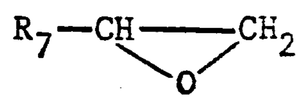 und anschliessender Acylierung mit einem Acylchlorid der Formel $R_8$Cl eingeführt werden. Analog kann ein Hydroxyalkyl-Rest eingeführt werden.

Als Zwischenprodukte verwendbare Verbindungen vom Typus des 2,2,6,6-Tetramethyl-4-(alkoxycarbonylcyanomethyl)-piperidins sind zudem aus der GB-PS 1.214.426 bekannt.

Die Einführung eines Restes $R_{22}$ geschieht ausgehend von den 4-Hydroxy- bzw. 4-Aminopiperidinen nach gebräuchlichen Methoden z.B. durch Umsetzung mit einem Chlorid der Formel $R_{26}Cl$, oder hydrogenolytische Kondensation mit $H_2N-R_{26}$.

Die Anforderung, welche an die an den angegebenen Stellen beschriebenen Gruppen der Formel IIa, IIb, Va, Vb und VI gestellt wird, ist, dass sie eine funktionelle Gruppe, insbesondere eine Amino-, Hydroxy- oder Carbonsäure(-ester)Gruppe enthalten, welche es ermöglicht, die erwähnten Gruppen mittels gebräuchlicher Umesterungs- bzw. Kondensationsreaktionen ins chelatbildende Molekül einzubauen.

Sind diese Gruppen z.B. in den Substituenten $R_{15}$ oder $R_{16}$ als mit einer Gruppe Va oder Vb, worin Z -O- ist, substituiertes Alkyl, so wird auf bekannte Weise ein Hydroxyphenylalkylchlorid mit einem von den Gruppen Va oder Vb abgeleiteten Alkohol, oder Amin in Gegenwart einer Base umgesetzt. Ebensogut eignet sich die Reaktion mit einem Alkoholat, welche unter dem Namen Williamson Synthese bekannt ist. Bedeutet in der Formel Vb Z -O-C(O)- oder ist $R_{15}$ oder $R_{16}$ eine Gruppe der Formel Vc, worin $R_{23}$ z.B. einen Rest VI bedeutet, so erfolgt deren Einführung am einfachsten dadurch, dass man mittels einer geläufigen Umesterungsreaktion aromatische Carbonsäuren, wie z.B. Benzoesäure, Phenylessigsäure oder β-Phenylpropionsäure, mit einem von der Formel Va oder Vb abgeleiteten Alkohol umsetzt. Diese Reaktion kann Säure- oder basenkatalysiert sein. Bedeutet $R_{15}$ oder $R_{16}$ eine mit einem Rest der Formel Vc substituierte Alkoxygruppe, so erfolgt deren Ein-

führung z.B. durch die Umsetzung eines Phenols, wie z.B. eines Brenzchatechins, Resorcins, Hydrochinons, Pyrogallols oder Phloroglucins mit einem Halogenalkylderivat der Gruppe der Formel Vc in einem inerten Lösungsmittel in Gegenwart einer Base. Bedeutet $R_{15}$ oder $R_{16}$ eine Gruppe der Formel VI, so wird ein Phenol mit einem 4-Oxo-Derivat einer Gruppe der Formel VI in Gegenwart einer Säure und anschliessender Reduktion umgesetzt, wie es z.B. auch in dem US-Pat. 3,847,930 beschrieben ist.

Ist L eine Gruppe der Formel IVa, so kann deren Herstellung analog der Beschreibung in dem US-Pat. 2,971,941 erfolgen, wobei ungefähr 2 Mole eines Phenols mit 1 Mol $SCl_2$ umgesetzt werden und die Einführung der Reste $R_{15}$ und $R_{16}$ wie oben beschrieben, vor oder nach der $SCl_2$-Stufe erfolgt. Bedeutet $R_{15}$ eine Gruppe der Formel VI, so wird diese vor der $SCl_2$-Stufe eingeführt. Die so hergestellten der Formel IVa entsprechenden Chelatbildner $H_2L$ sind neu und bilden daher einen Gegenstand der vorliegenden Erfindung. Verbindungen, welche eine Gruppe der Formel IVa enthalten, zeichnen sich dadurch aus, dass r einen Wert von 1 oder 2 besitzen kann. Dieser ist abhängig von dem Verhältnis der Einsetzungskonzentration des freien Liganden und des Metallsalzes im oben beschriebenen Chelatisierungsschritt.

Ist L eine Gruppe der Formel IVb, so kann deren Herstellung analog wie für die Verbindungen aus dem GB-Pat. 878,362 oder dem GB-Pat. 1,332,560 erfolgen. Enthalten $R_{15}$ oder $R_{16}$ eine Gruppe IIa oder IIb, respektive Va, Vb oder VI, so werden diese wie oben beschrieben eingeführt. Bedeutet $R_{17}$ eine Gruppe der Formel Vc, worin $R_{23}$ einen Rest der Formel VI bedeutet, so geht man von den bekannten Verbindungen, worin

$R_{23}$ Alkyl bedeutet aus, und estert diese mit einem von der Gruppe Va abgeleiteten Alkohol auf bekannte Weise um. Ist $R_{17}$ eine Gruppe der Formel $-OR_{24}$, so setzt man am einfachsten die analoge Verbindung, worin $R_{17}$ Hydroxy ist, mit einer Verbindung $ClR_{24}$ um. Einige der Chelatbildner der Formel IVb sind aus der JA-OS 51-88484 bekannt.

Ist L eine Gruppe der Formel IVc, so werden, falls $R_{15}$ oder $R_{16}$ eine Gruppe IIa oder IIb, bzw. Va, Vb oder VI enthalten, diese wie oben beschrieben eingeführt.

Bedeuten $R_{18}$ und $R_{19}$ zusammen =O, so dienen in der Regel als Ausgangsstoffe Salicylsäure bzw. deren Alkylester, Salicylamid oder ein α-Hydroxyphenylketon. Die Einführung einer Gruppe der Formel Va als $R_{20}$ erfolgt daher durch eine einfache Umesterungsreaktion ausgehend von einem Salicylsäureester mit einem Alkohol oder Amin, welches von der Formel Va abgeleitet wird. Auf analoge Weise können Verbindungen durch Umesterung hergestellt werden, in denen $R_{20}$ eine durch eine Gruppe der Formel Vc substituierte Alkyl-, Alkenyl-, Cycloalkyl- oder Arylgruppe bedeutet. Um zu den Verbindungen zu gelangen, in welchen $R_{20}$ eine Gruppe der Formel VII bedeutet, werden ungefähr 2 Mole eines Salicylsäureesters mit ungefähr 1 Mol eines Diols umgesetzt. Vor oder nach dieser Stufe können die gewünschten Reste $R_{15}$ und $R_{16}$ auf oben angegebene Weise eingebaut werden. Die so erhaltenen Chelatbildner der Formel IVc, worin $R_{18}$ und $R_{19}$ zusammen =O sind, sind neue Verbindungen und bilden daher einen Gegenstand der vorliegenden Erfindung.

Aus den so erhaltenen Keto-Verbindungen können auf bekannte

Weise die Chelatbildner der Formel IVc, worin $R_{18}$ und $R_{19}$ zusammen $=NR_{27}$ bedeuten hergestellt werden. Dabei werden die Keto-Verbindungen in einer geläufigen Kondensationsreaktion mit einem primären Amin umgesetzt. Es kann sich dabei um ein Amin handeln, welches von einer Gruppe der Formel Va, worin $X_1$ -NH bedeutet, abgeleitet ist, oder es kann auch ein mit einer Gruppe der Formel Vc substituiertes Alkyl-, Alkenyl-, Cycloalkyl- oder Arylamin sein. Die erhaltenen Verbindungen der Formel IVc, worin $R_{18}$ und $R_{19}$ zusammen $=NR_{27}$ bedeuten sind neue Stoffe und bilden daher auch einen Gegenstand der vorliegenden Erfindung. Zu den Verbindungen, worin $R_{27}$ eine Gruppe der Formel VIIIb ist gelangt man, indem man das primäre Amin durch ein Diamin ersetzt und mit 2 Molen der Ketoverbindung reagieren lässt.

Die beschriebenen Azomethin-Verbindungen der Formel IVc können durch bekannte Reduktionsverfahren, z.B. mittels Wasserstoff auf Pd/C, zu den Verbindungen der Formel IVc reduziert werden, worin $X_1$ -NH bedeutet. Eine andere Variante ist, in einer Mannich-Reaktion in Gegenwart von einem Aldehyd, vorzugsweise Formaldehyd, ein sekundäres Amin $HN(R_{25})R_{27}$ einzusetzen. Enthält der Chelatbildner als $R_{27}$ eine Gruppe der Formel VIIIa, so erfolgt die Herstellung durch Reduktion der entsprechenden Di-Azomethin-Verbindung. Die auf die beschriebene Weise erhaltenen Verbindungen der Formel IVc, worin $R_{18}$ $-N(R_{25})R_{27}$ bedeutet, sind neu, und stellen daher auch einen Erfindungsgegenstand dar.

Verwendet man als Liganden solche, welche ein $r = 2$ besitzen, so können sich Oligomere oder gar Polymere bilden. Diese zeichnen sich dadurch aus, dass sie mehrere chelatbildungsfähige Zentren enthalten und haben daher die Möglichkeit

mehrere Metallzentren zu binden. Solche höhermolekularen Substanzen zeichnen sich durch eine erhöhte Extraktionsbeständigkeit aus. Solche höhermolekulare Stoffe sind neu, und sind daher ebenfalls Gegenstand der Erfindung, was vor allem dadurch zum Ausdruck kommt, dass das in der Formel I gegebene Verhältnis von Metall zu Ligand L auch in diesem Falle stimmt, falls es auf den dem Polymeren zugrundeliegenden Baustein berechnet wird. Das Molekulargewicht beträgt ungefähr 400-10000, bevorzugt 400-2000.

Die Verbindungen der Formel I können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DT-OS 2.456.864 auf den Seiten 12-14 aufgeführten Polymeren.

Von besonderer Bedeutung ist die Stabilisierung von Polyolefinen, Styrolpolymerisaten und von Polyurethanen, für die sich die Verbindungen der Formel I hervorragend eignen. Beispiele hierfür sind Polyäthylen hoher und niedriger Dichte, Polypropylen, Aethylen-Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Filmen, Fasern, Lacken, Elastomeren oder Schaumstoffen.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabiliserenden Kunststoffen zugesetzt werden.

Im Falle von vernetztem Polyäthylen werden die Verbindungen vor der Vernetzung beigefügt.

Die Erfindung betrifft daher auch die durch Zusatz von 0,01 bis 5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

Antioxydantien, wie einfache 2,6-Dialkylphenole, Derivate von alkylierten Hydrochinonen, hydroxylierte Thiodiphenyläther, Alkyliden-bisphenole, O-, N- und S-Benzylverbindungen, hydroxybenzylierte Malonester, Hydroxybenzyl-Aromaten, s-Triazinverbindungen, Amide der β-(3,5-Di-tert.-butyl-4-hydroxyphenyl)-propionsäure, Ester der β-(3,5-Di-tert.-butyl-4-hydroxyphenyl)-propionsäure, Ester der β-(5-tert.-Butyl-4-hydroxy-3-methylphenyl)-propionsäure, Ester der 3,5-Di-tert.-butyl-4-hydroxyphenylessigsäure, Acylaminophenole, Benzylphosphonate, Aminoarylderivate, UV-Absorber und Lichtschutzmittel, wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate; des weiteren Nickelverbindungen, sterisch gehinderte Amine, Oxalsäurediamide, Metalldesaktivatoren, Phosphite, Peroxidzerstörende Verbindungen, Polyamidstabilisatoren, basische Co-Stabilisatoren, PVC-Stabilisatoren, Nukleierungsmittel oder sonstige Zusätze wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in DT-OS 2.427.853 auf Seiten 18-24.

Die Herstellung und Verwendung der erfindungsgemässen Verbindungen wird in den folgenden Beispielen näher beschrieben.

Beispiel 1

137,2 g (0,5 Mol) N-[2-Hydroxybenzyl]-N-methyl-N-[2,2,6,6-tetramethyl-piperidyl-4]-amin wurden in 200 ml Aethanol gelöst, und bei Raumtemperatur während 1,5 Stunden eine Lösung aus 11,5 g Na in 300 ml Aethanol zugetropft und während weiteren 1,5 Stunden bei 50°C gerührt. 59,4 g $NiCl_2 \cdot 2H_2O$, gelöst in 300 ml Aethanol wurde während 2 Stunden zugetropft und anschliessend die Lösung für 2 Stunden am Rückfluss gehalten. Das Lösungsmittel wurde abgedampft, der Rückstand in heissem Hexan aufgenommen und das NaCl abfiltriert. Nach dem Abkühlen der Hexanlösung kristallisierte der Stabilisator 1 als grüngelb gefärbte Verbindung aus.

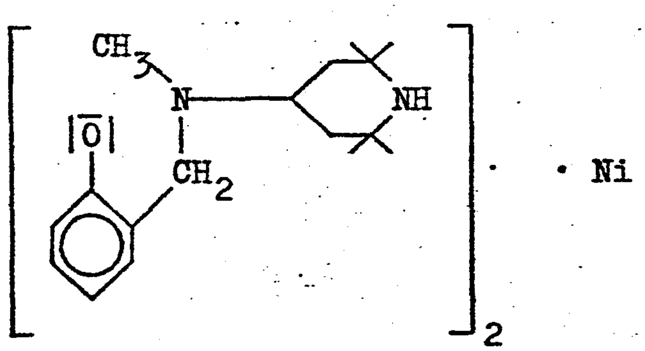

| | |
|---|---|
| Ni berechnet (bezüglich $H_2O$) | 9,26% |
| Ni gefunden | 9,67% |
| gefunden (bezüglich $H_2O$) | 4,5% |

Analog Verbindung I wurden die folgenden Verbindungen hergestellt.

| Beispiel | Stabilisator | Berechnet Ni (bezügl. $H_2O$) % | Gefunden Ni % | $H_2O$ % |
|---|---|---|---|---|
| 2 | 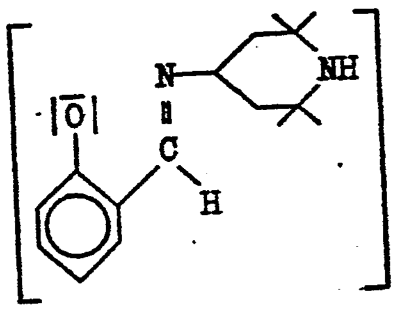 Ni 2 | 10,00 | 9,2 | 1,3 |
| 3 | 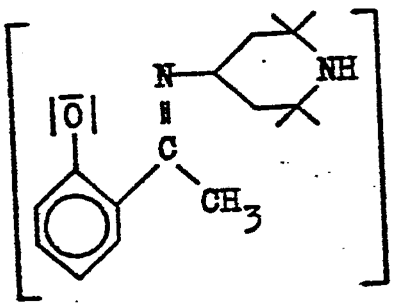 Zn 2 | 10,26 | 9,9 | 3,9 |
| 4 | 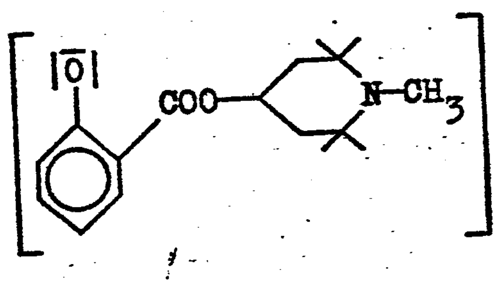 Ni 2 | 9,14 | 8,57 | 0,6 |
| 5 | 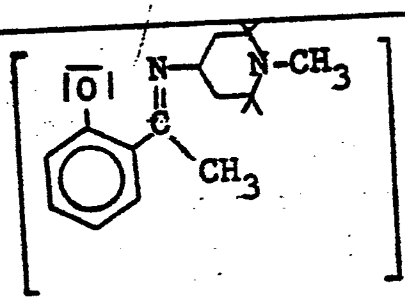 Ni 2 | 8,8 | 7,96 | 4,2 |
| 6 | 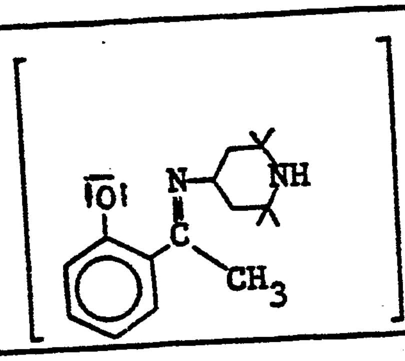 2 Ni | 9,7 | 9,67 | 4,4 |

Beispiel 7



15,66 g Di-{2-hydroxy-5-t-octyl-3-[N-methyl-N-(2,2,6,6-tetramethylpiperidyl-4)-aminomethyl]-phenyl} sulfid wurden in 100 ml Aethanol gelöst und unter Stickstoffatmosphäre wurden 1,36 g Natriumäthanolat zugefügt. Die Reaktionsmischung wurde während 20 Minuten bei Raumtemperatur gerührt und anschliessend 1,65 g $NiCl_2 \cdot 2H_2O$ gelöst in 40 ml Aethanol zugetropft. Die Mischung wurde während 12 Stunden bei 24°C gerührt. Das ausgefallene NaCl wurde abfiltriert, und das Lösungsmittel abgedampft. Die so erhaltenen 16,9 g des blassgrünen Stabilisators 7

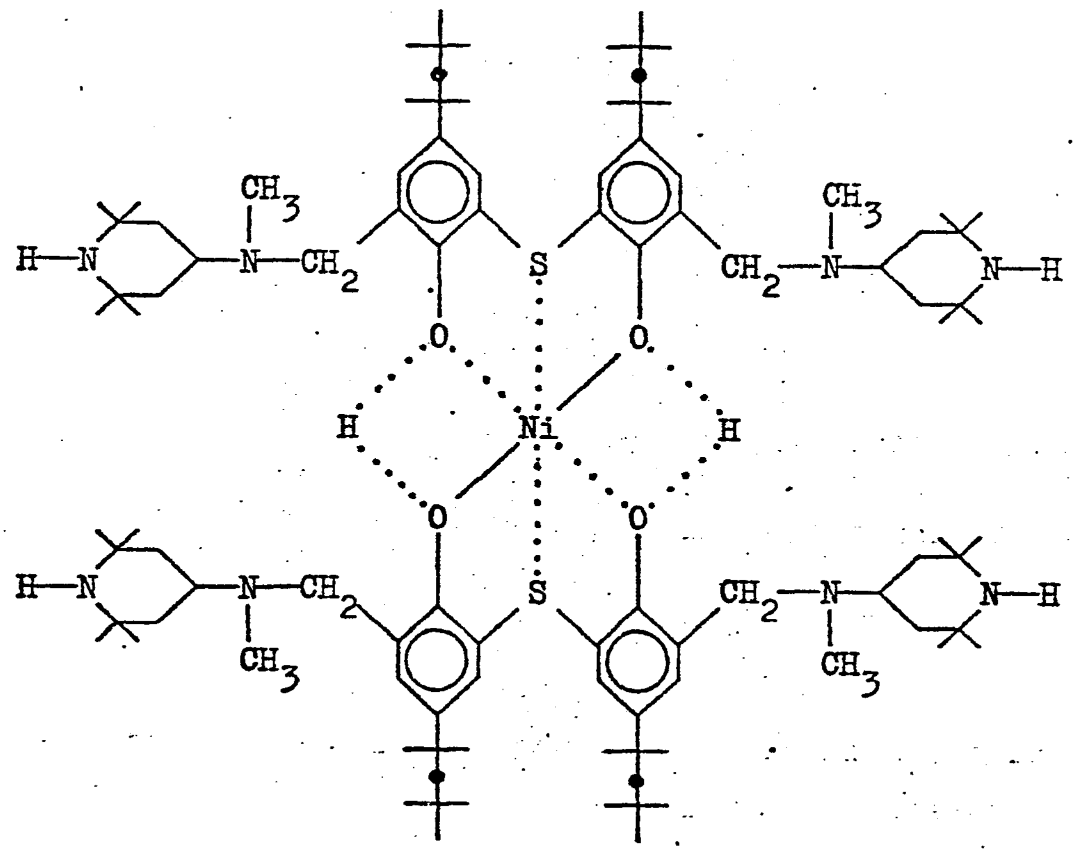

Stabilisator 7

zeigte folgende Analyse:

$C_{100}H_{170}N_8O_4S_2 \cdot Ni \cdot 2H_2O$

| | |
|---|---|
| Ni berechnet (bezüglich $H_2O$) | 3,51% |
| Ni gefunden | 3,43% . |

Beispiel 8

12,6 g [0,02 Mol] des Ni-Salzes des N-Methyl-N-[2,2,6,6-tetramethyl-piperidyl-4)-amin und 4 g (0,02 Mol) des N,N-Dimethyl-N-[1,2,2,6,6-pentamethylpiperidyl-4]-amins wurden in 200 ml Toluol gelöst und für 7 Stunden über dem Wasserabscheider am Rückfluss gehalten. Danach wurde das Lösungsmittel abgedampft, der Rückstand in heissem Hexan aufgenommen und nach dem Abkühlen die gebildeten Kristalle abgesaugt und im Vakuumofen bei 60°C für 20 Stunden getrocknet. Das Produkt

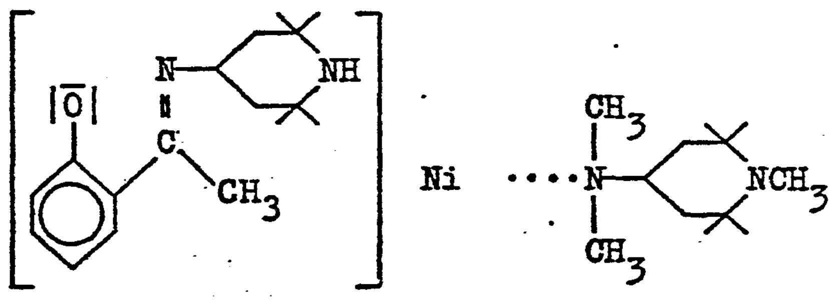

Stabilisator 8

zeigte folgende Analyse:

Ni berechnet (bezüglich $H_2O$) 7,03%
Ni gefunden 7,65%.

Analog Stabilisator 8 wurden die folgenden Verbindungstypen hergestellt:

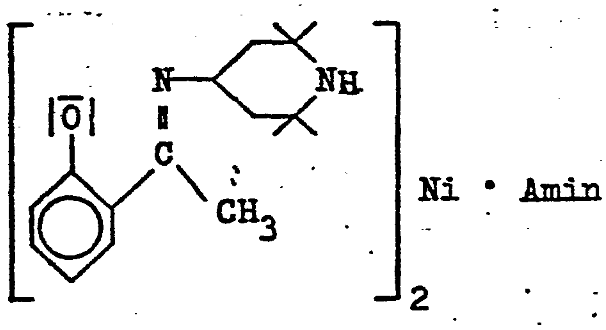

Die entsprechenden als Amine verwendeten Liganden A sind in den Beispielen der folgenden Tabelle zusammengestellt.

| Beispiel | Amin A | Berechnet Ni (bezügl. $H_2O$) % | Gefunden Ni % | $H_2O$ % |
|---|---|---|---|---|
| 9 | 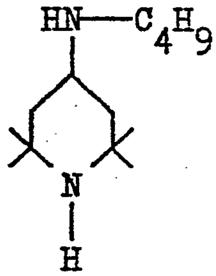 | 6,9 | 6,69 | 3,7 |
| 10 | 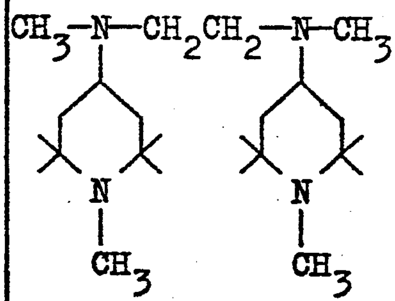 | 5,69 | 6,13 | 3,0 |
| 11 | 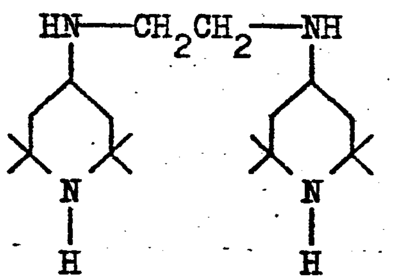 | 6,03 | 6,45 | 2,9 |

Analog wie der Stabilisator gemäss Beispiel 8 wurden die folgenden Verbindungen hergestellt:

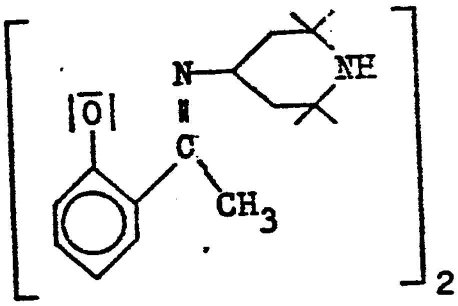

Zn · Amin

| Beispiel | Amin A | a) Berechnet Ni<br>b) Berechnet N<br>(bezügl. $H_2O$)<br>% | a) Gefunden Ni<br>b) Gefunden N<br>% | $H_2O$<br>% |
|---|---|---|---|---|
| 12 | 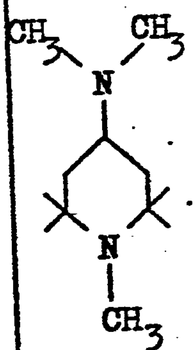 | a) 7,86 | a) 7,09 | 2,6 |
| 13 | 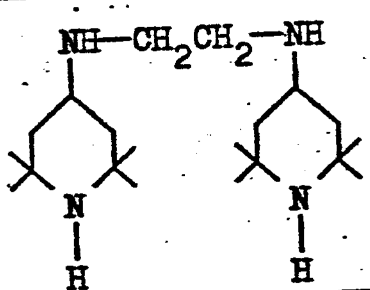 | a) 6,73<br>b) 11,54 | a) 5,78<br>b) 11,4 | 2, |
| 14 | 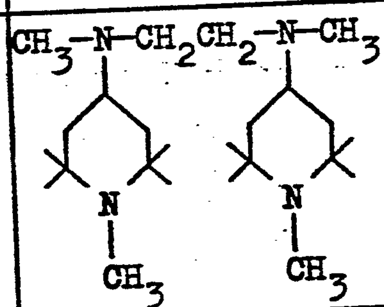 | a) 6,36 | a) 5,76 | 2, |
| 15 | 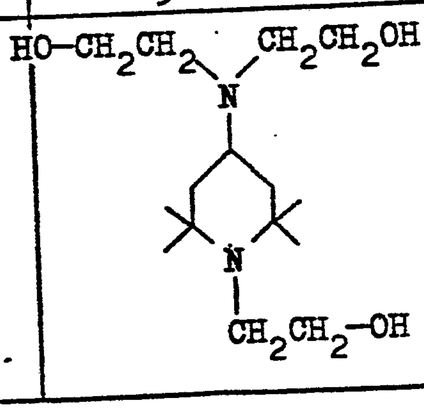 | a) 7,51<br>b) 9,66 | a) 5,85<br>b) 9,13 | 2. |

Analog wie Stabilisator gemäss Beispiel 8 wurden folgende Verbindungen hergestellt:

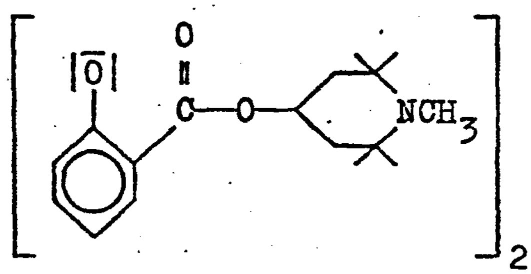

Ni · Amin

| Beispiel | Amin A | Berechnet Ni (bezügl. $H_2O$) % | Gefunden Ni % | $H_2O$ % |
|---|---|---|---|---|
| 16 | 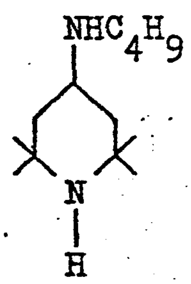 | 6,89 | 6,51 | 0,5 |
| 17 | 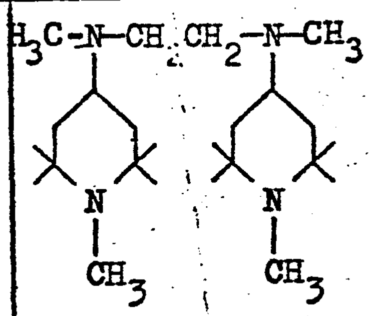 | 4,61 | 5,11 | <0,3 |
| 18 | 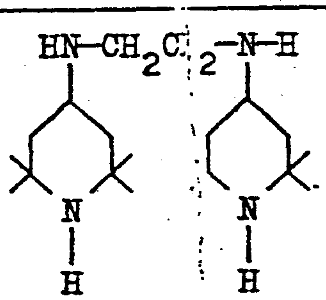 | 5,98 | 5,29 | 0,6 |

Beispiel 19

a) 22,1 g 3,3'-Di-(t-octyl)-6,6'-diphenylsulfid und 26,8 g 4-n-Octylamino-2,2,6,6-tetramethylpiperidin wurden in 100 ml Aethanol aufgelöst. Unter Stickstoff wurden 8,6 g einer 35%-igen Lösung von Formaldehyd bei 23°C zugetropft. Die Reaktionsmischung wurde während 4 h bei Rückflusstemperatur gehalten und das Lösungsmittel anschliessend unter Vakuum abgedampft, was 35,2 g des Di-{2-Hydroxy-5-t.octyl-3-[N-octyl-N-(2,2,6,6-tetramethylpiperidyl-4)-aminomethyl ] phenyl} -sulfids ergab.

b) 20,08 g des Produkts aus Beispiel 19a wurde analog Beispiel 5 mit Natriumäthanolat (aus 0,92 g Na in 20 ml Aethanol) und 3,3 g $NiCl_2 \cdot 2H_2O$ behandelt. Das so erhaltene Produkt: 21,3 g Stabilisator 19

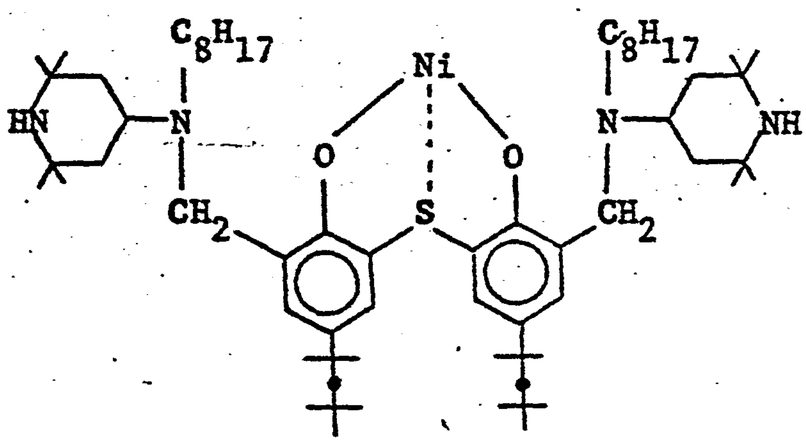

welcher folgende Analysenwerte aufwies:

$C_{64}H_{112}N_4O_2S \cdot Ni \cdot 2H_2O$

| | | |
|---|---|---|
| Ni (berechnet) | : | 5,35 % |
| Ni (gefunden) | : | 5,1 % |

Beispiel 20

13,3 g des Stabilisators 19 wurden in 150 ml Aethanol aufgenommen und unter Stickstoffatmosphäre bei 24°C mit 1,87 g 4-Amino-2,2,6,6-tetramethylpiperidin in 20 ml Aethanol tropfweise versetzt, und die Reaktionslösung während 16 h gerührt. Das Lösungsmittel wurde danach abgedampft, was 15,0 g grüner Kristalle ergab (Stabilisator 20).

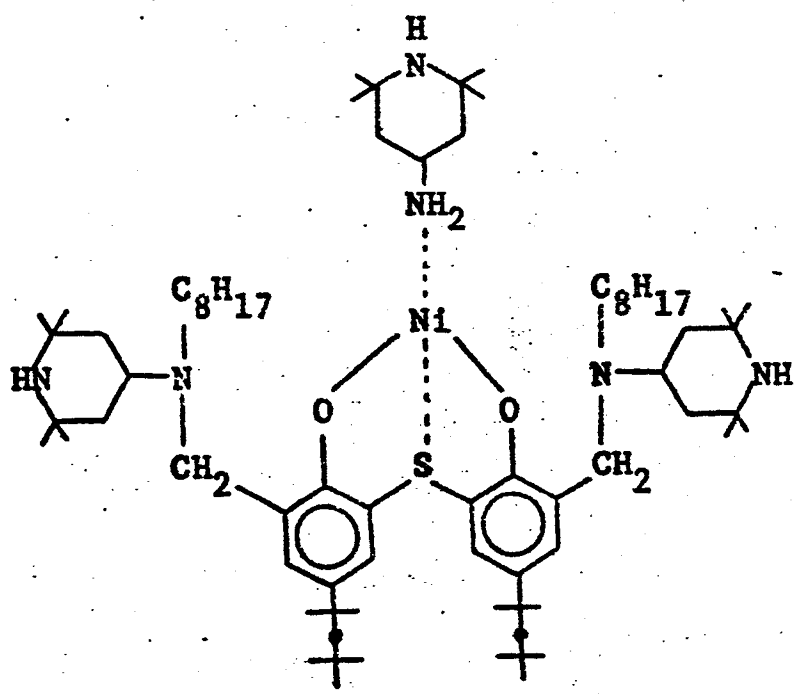

welche folgende Analysenwerte besassen:

| | | |
|---|---|---|
| Ni (berechnet) | : | 4,83 % |
| Ni (gefunden ) | : | 4,54 % |

Beispiel 21

19,7 g (N-[2-Hydroxy-4-t.octylbenzyl]-N-methyl-N-[2,2,6,6-tetramethyl-piperidinyl-4]-amin in 150 ml Methanol wurden mit frisch hergestelltem Natriummethylat (aus 1,15 g Na und 40 ml Methanol) versetzt. Nach 1/2-stündigem Rühren wurden 4,3 g $NiCl_2 \cdot 2H_2O$ in 50 ml Methanol zugetropft und das Rühren für 5 h bei 24°C fortgesetzt. Das Natrium wurde abfiltriert und das Lösungsmittel abgedampft. Der grün-braune Rückstand

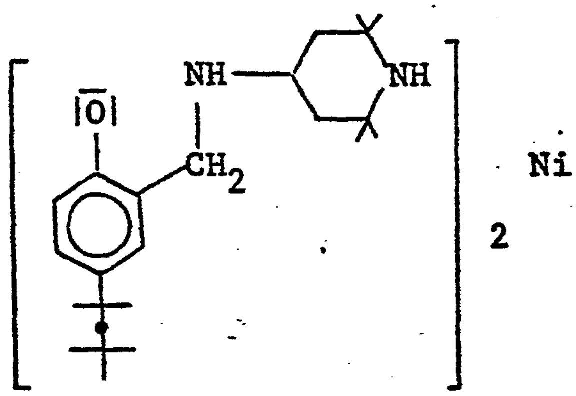

Stabilisator 21 besitzt folgende Analysendaten:

| | | | |
|---|---|---|---|
| Ni | (berechnet) | : | 7,04 % |
| Ni | (gefunden) | : | 7,28 %. |

Beispiel 22

52,5 g (0,1 Mol) N-[2-Hydroxybenzyl]-N-[2,2,6,6-tetramethyl-piperidyl-4]-amin wurden in 250 ml Methanol gelöst und mit 100 ml einer 1 molaren Natriummethylatlösung versetzt. In diese Lösung wurden innerhalb von 1/2 Stunden bei Raumtemperatur 25 ml einer 2 molaren Lösung von wasserfreiem Nickelchlorid in Methanol (entsprechend 0,05 Mol $NiCl_2$)

zugetropft und das Reaktionsgemisch während 30 Minuten unter Rückfluss gekocht. Das abgeschiedene Natriumchlorid wurde abfiltriert und das Filtrat zur Trockene eingeengt. Der Rückstand wurde mit Aether bei Raumtemperatur extrahiert, das Extrakt eingedampft und bei 80° unter einem Druck von 11 mm Hg getrocknet. Man erhielt ein dunkelbeiges Pulver der Zusammensetzung:

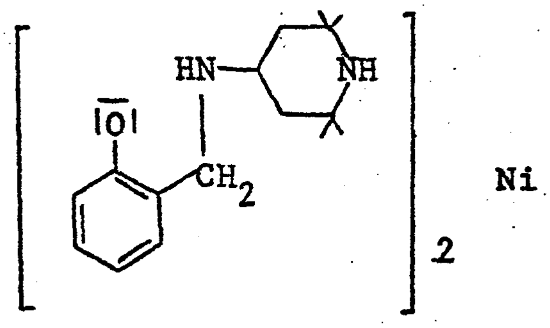

(Stabilisator 22)

| | | | | | |
|---|---|---|---|---|---|
| Ni | (berechnet) | 10,10 % | Ni | (gefunden) | 10,03 % |
| C | " | 66,10 % | C | " | 65,94 % |
| H | " | 8,67 % | H | " | 8,85 % |
| N | " | 9,63 % | N | " | 9,71 % |

## Beispiel 23

52,5 g (0,1 Mol) N-[2-Hydroxybenzyl]-N-[2,2,6,6-tetramethylpiperidyl-4]-amin wurden in 250 ml absolutem Aethanol gelöst und mit 100 ml einer 1 molaren Natriummethylatlösung versetzt. In diese Lösung tropfte man eine Lösung von 6,81 g (0,05 Mol) wasserfreiem Zinkchlorid in 20 ml absolutem Aethanol, erhitzte das Gemisch 1 1/2 Stunden unter Rückfluss und dampfte das Lösungsmittel anschliessend ab. Der Rückstand wurde darauf mit heissem Toluol extrahiert, das Extrakt eingedampft und bei 60° unter einem Druck von 11 mm Hg getrocknet. Man erhielt dabei ein gelbliches Pulver folgender Zusammensetzung:

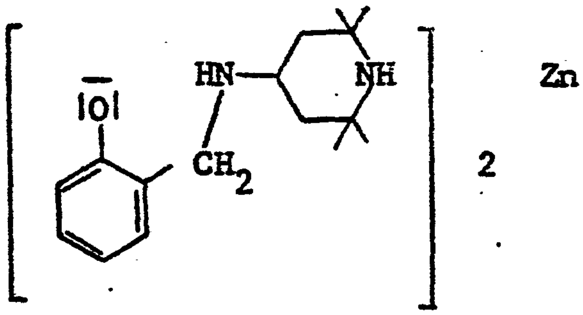 Stabilisator 23

| | | | | | |
|---|---|---|---|---|---|
| Zn | (berechnet) | 11,11 % | Zn | (gefunden) | 10,5/10,4 % |
| C | " | 65,35 % | C | " | 64,42/64,39% |
| H | " | 8,57 % | H | " | 8,76/8,56 % |
| N | " | 9,53 % | N | " | 9,07/9,14 % |

Beispiel 24

Lichtschutzwirkung in Hochdruckpolyäthylen-Folien

100 Teile Polyäthylengranulat niederer Dichte (= 0,917) werden mit 0,05 Teilen eines Stabilisators der folgenden Tabelle im Brabenderplastographen bei 180°C während 10 Minuten homogenisiert. Die so erhaltene Masse wird möglichst rasch dem Kneter entnommen und in einer Kniehebelpresse zu einer 2 - 3 mm dicken Platte gepresst. Ein Teil des erhaltenen Rohpresslings wird ausgeschnitten und zwischen zwei Hochglanz-Hartaluminiumfolien mit einer handhydraulischen Laborpresse während 6 Minuten bei 170° und 12 Tonnen Druck zu einer 0.1 mm dicken Folie gepresst, die unverzüglich in kaltem Wasser abgeschreckt wird. Aus dieser werden nun Abschnitte von je 60 x 44 mm gestanzt und im Xenotest 1200 belichtet. Zu regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei der Belichtung ist ein Mass für den photooxidativen Abbau des Polymeren (s.L.Blaban et al., J.Polymer Sci. Part C, 22,

1059 - 1071 (1969); J.F.Heacock, J.Polymer Sci. Part A-1, 22, 2921-34 (1969); D.J. Carlsson and DM. Wiles, Macromolecules 2, 587-606 (1969) und ist erfahrungsgemäss mit einem Abfall der mechanischen Eigenschaften des Polymeren verbunden.

Als Mass der Schutzwirkung gilt die Zeit bis Erreichen einer Carbonylextinktion von 0,100.

Die Ergebnisse sind in der Tabelle I zusammengefasst.

Tabelle I

| Additiv | Belichtungszeit bis CO-Extinktion 0,1 |
|---|---|
| keines | 270 h |
| Nr. 3 | 1630 h |
| Nr. 10 | 1630 h |
| Nr. 12 | 2100 h |
| Nr. 11 | 1800 h |

**Beispiel 25**

100 Teile Polypropylenpulver (Schmelzindex 1,5 g/10 Min., 230°C, 2160 g Belastung) wurden in einem Trommelmischer mit 0,1 Teil Pentaerythrit-tetrakis [3-(3'5'-ditertiär-butyl-4-hydroxyphenyl)-propionat] und 0,15 Teile der in der nachfolgenden Tabelle angeführten Stabilisatoren gemischt und in einem Extruder bei 200 - 220°C extrudiert und granuliert. Das erhaltene Granulat wurde in üblicher Weise mittels eines Extruders mit Breitschlitzdüse zu Folien verarbeitet, die in Bändchen geschnitten wurden,

welche anschliessend bei erhöhter Temperatur auf die sechsfache Länge verstreckt wurden. Der Titer dieser Bändchen beträgt 700 - 900 den, ihre Breite 4 mm, ihre Reissfestigkeit 5,5 - 6,5 g/den.

Diese Bändchen wurden in einem Xenotest 1200 belichtet. In regelmässigen Abständen wurden Proben einem Zug-Dehnungsversuch unterworfen, wobei sich mit zunehmender Belichtung eine fortschreitende Abnahme der Reissfestigkeit ergibt. Die Belichtungszeit bis zum Abfall der Reissfestigkeit auf die Hälfte des Ausgangswertes wird durch die Wirkung der Lichtschutzmittel verlängert.

| Stabilisator Nr. | Belichtungszeit im Xenotest 1200 bis die Reissfestigkeit auf die Hälfte des Ausgangswertes gesunken ist: |
|---|---|
| keiner | 400 h |
| 14 | 2300 h |
| 16 | 2030 h |
| 20 | 3950 h |
| 12 | 2100 h |
| 11 | >2000 h |
| 10 | 3080 h |
| 15 | 2100 h |
| 8 | 2050 h |
| 9 | 910 h |
| 5 | 3680 h |

## Beispiel 26

Lichtschutzwirkung in PP-Fasern (130/37)

1000 Teile unstabilisiertes Polypropylenpulver (Schmelzindex ~18) werden in einem Trommelmischer mit 1 Teil Pentaerithrytyl-tetrakis[3-(3,5-di-tert-butyl-4-hydroxylphenyl)-propionat] und mit 3 Teilen der in der Tabelle angeführten Lichtschutzmittel gemischt und anschliessend in einem Extruder bei 220°C extrudiert und granuliert. Das erhaltene Granulat wird in einer Labor-Schmelzspinnanlage bei einer maximalen Temperatur von 270°C und einer Geschwindigkeit von 300 m/Minute zu einem 130/37 denier Multifilament versponnen. Dieses wird verstreckt und gezwirnt mittels einer Streckzwirnmaschine. Das Streckverhältnis ist 1:5,6 und die Zwirnzahl 15/Meter, so dass schliesslich Multifilamente 130/37 denier erhalten werden. Diese Multifilamente werden auf weissen Karton montiert und im Xenotest 1200 belichtet.

Als Mass der Schutzwirkung gilt die Belichtungszeit bis 50 % Reissfestigkeitsverlust.

Die Ergebnisse sind in der Tabelle zusammengefasst:

| Stabilisator Nr. | Belichtungszeit im Xenotest 1200 bis die Reissfestigkeit auf die Hälfte des Anfangswerts gesunken ist |
|---|---|
| keiner | 250 h |
| 3 | 500 h |
| 4 | 1400 h |
| 6 | 825 h |

## Patentansprüche

1. Neue Metallkomplexe der allgemeinen Formel I

$$\left[M^{q\oplus}\right] \left[L^{r\ominus}\right]_{(q-s)/r} \left[B^{\ominus}\right]_{s} \cdot m\,A \qquad (I)$$

worin

M ein zweifach oder dreifach positiv geladenes Metallion ist,

q 2 oder 3 ist, und

L ein 1 oder 2 Phenolatgruppen enthaltendes t-zähniges chelatbildendes Anion ist, welches mindestens eine Gruppe der Formeln IIa oder IIb

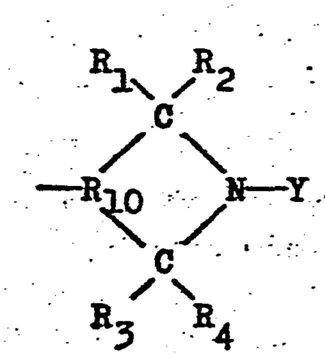 (IIa) 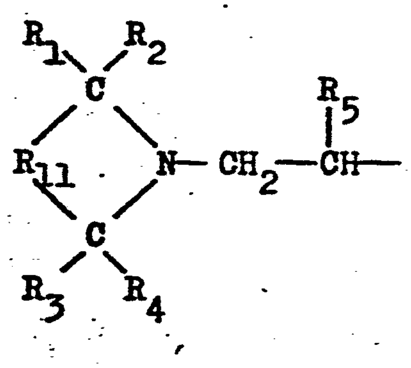 (IIb)

enthält, worin

t 2, 3 oder 4 ist, und

$R_1, R_2, R_3$ und $R_4$ unabhängig voneinander Alkyl sind, oder

$R_1$ und $R_3$ zusammen Alkylen sind, oder

$R_1$ und $R_2$ bzw. $R_3$ und $R_4$ unabhängig voneinander zusammen Alkylen oder Azaalkylen sind, und

$R_5$ Wasserstoff, Methyl oder Phenyl bedeutet, und

Y Wasserstoff, Oxyl, Alkyl, Alkenyl, Alkinyl, Alkoxyalkyl, Aralkyl, 2,3-Epoxypropyl, eine aliphatische Acylgruppe oder eine der Gruppen $-CH_2COOR_6$,

$-CH_2-CH(R_7)-OR_8$, $-COOR_9$ oder $-CONHR_9$ ist, worin

$R_6$ Alkyl, Alkenyl, Phenyl, Aralkyl oder Cycloalkyl ist und

$R_7$ Wasserstoff, Methyl oder Phenyl ist, und

$R_8$ Wasserstoff, eine aliphatische oder aromatische, eine araliphatische oder alicyclische Acylgruppe bedeutet, worin der aromatische Teil gegebenenfalls mit Chlor, Alkyl, Alkoxy und/oder Hydroxy substituiert sein kann, und

$R_9$ Alkyl, Cyclohexyl, Phenyl oder Benzyl bedeutet, und

$R_{10}$ ein dreiwertiger und

$R_{11}$ ein zweiwertiger organischer Rest ist, wobei

$R_{10}$ und $R_{11}$ den N-haltigen Ring zu einem 5-, 6- oder 7-gliedrigen Ring ergänzen, und

r gleich der Anzahl koordinativ gebundenen Phenolatanionen im Molekül 1 oder 2 ist, und

B ein einfach negativ geladener Ligand ist, und

s eine ganze Zahl von 0 bis 2 ist, wobei die Summe $(q-s)/_r + s = q$ sein muss, und

m eine ganze Zahl von 0 bis 3 ist, wobei die Summe $t \cdot [(q-s)/_r] + s + m$ gleich der Koordinationszahl des Metallions M ist, wobei

A $H_2O$ oder ein Amin der Formel III

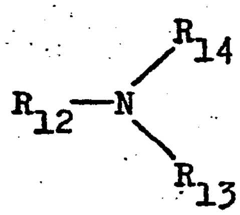

(III)

ist, worin

$R_{12}$ gegebenenfalls substituiertes Alkyl, Cycloalkyl, Aryl, Aralkyl, eine gegebenenfalls substituierte Aminoalkylgruppe oder eine Piperidinylgruppe ist, und

$R_{13}$ Wasserstoff oder gegebenenfalls substituiertes Alkyl, Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden, und

$R_{14}$ Wasserstoff oder gegebenenfalls substituiertes Alkyl bedeutet.

2. Neue Metallkomplexe gemäss Anspruch 1 der Formel I in denen $L^{r-}$ ein 2-, 3- oder 4-zähniger Chelatbildner ausgewählt aus der Klasse bestehend aus den Gruppen IVa, IVb und IVc

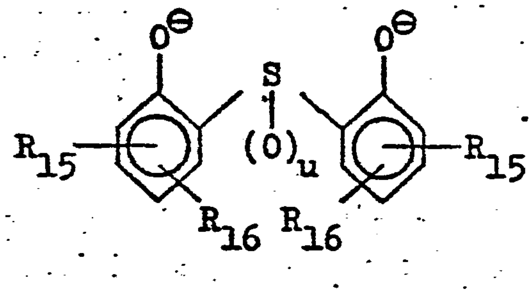

(IVa)

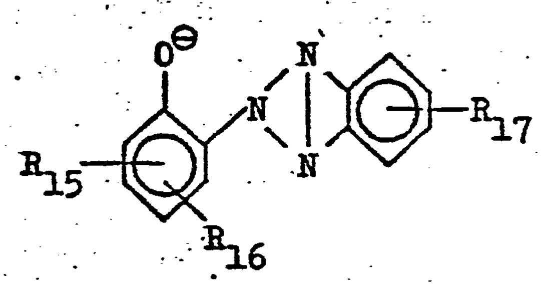

(IVb)

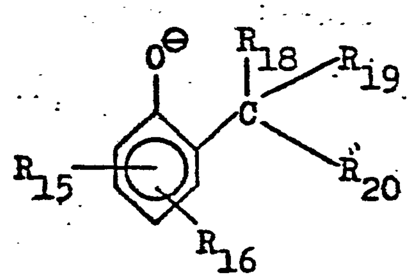

(IVc)

ist, worin

u	0, 1 oder 2 ist, und r oben angegebene Bedeutung hat, und

$R_{15}$ und $R_{16}$	unabhängig voneinander Wasserstoff, $C_1$-$C_{18}$ Alkyl, welches gegebenenfalls mit einer Gruppe Va, Vb oder Vc:

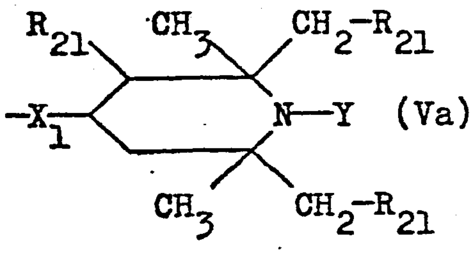 (Va), 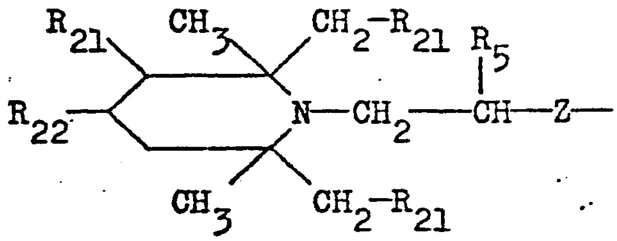 (Vb)

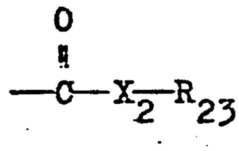 (Vc)

substituiert sein kann; oder eine Gruppe Vc, oder eine p-ständige Gruppe der Formel VI

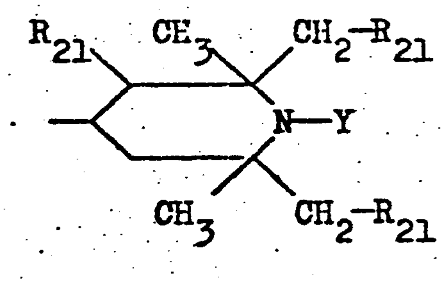 (VI) ,

Halogen oder einen Rest $-OR_{24}$ bedeuten oder $R_{15}$ und $R_{16}$ zusammen einen 1,3-Butadien-1,4-diyl-Rest bilden, wobei

$R_5$ und Y	die oben angegebene Bedeutung haben, und

$X_1$ und $X_2$	unabhängig voneinander -O- oder $-NR_{25}$ sind, und

$R_{21}$	Wasserstoff oder $C_1$-$C_8$ Alkyl ist, und

$R_{22}$	Wasserstoff oder eine Gruppe $-X_1R_{26}$ bedeutet, wobei $X_1$ die oben angegebene Bedeutung hat, und

Z	-O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, und

$R_{24}$ Wasserstoff, gegebenenfalls mit einer Gruppe der Formel Vc substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{17}$ Wasserstoff, $C_1$-$C_{18}$ Alkyl, Halogen, eine Gruppe $-OR_{24}$ oder eine Gruppe der Formel Vc bedeutet, und

$R_{18}$ eine Gruppe $-N(R_{25})R_{27}$ ist und

$R_{19}$ Wasserstoff ist, oder

$R_{18}$ und $R_{19}$ zusammen =O oder $=NR_{27}$ sind, und

$R_{20}$ Wasserstoff, $C_7$-$C_{14}$ Aralkyl, $C_7$-$C_{14}$ Alkylphenyl, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{10}$ Aryl ist, oder, falls $R_{18}$ und $R_{19}$ zusammen =O sind eine Gruppe der Formel Va ist; oder $R_{20}$ [illegible] der Formel [illegible]

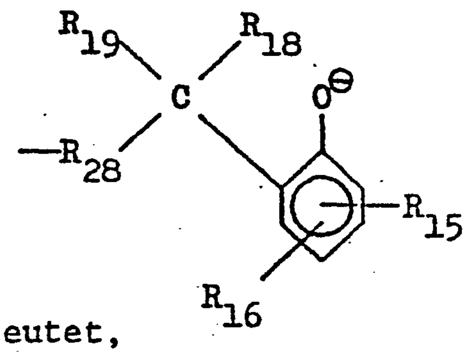

(VII) ,

bedeutet,
worin $R_{15}$, $R_{16}$, $R_{18}$ und $R_{19}$ oben angegebene Bedeutung haben, und

$R_{28}$ $C_1$-$C_{12}$ Alkylen, Butenylen, $C_6$-$C_{10}$ Arylen oder Diphenylen ist, bedeutet, und

$R_{27}$ Wasserstoff, $C_7$-$C_{14}$ Aralkyl, $C_7$-$C_{14}$ Alkylphenyl, Hydroxy, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder eine Gruppe der Formel VI, oder eine Gruppe der Formel $-CH_2-CH(R_5)-OR_{26}$; oder falls $R_{20}$ nicht eine Gruppe der Formel VII ist, $R_{27}$ auch eine Gruppe der Formel VIIIa oder VIIIb

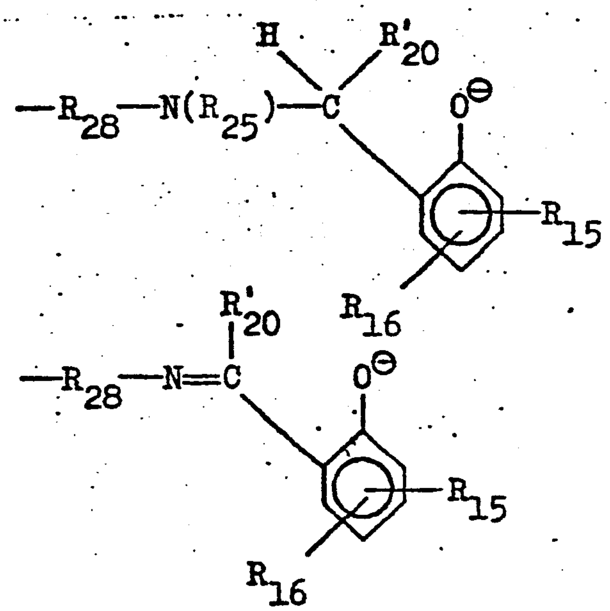

(VIIIa)

(VIIIb)

bedeutet, worin $R'_{20}$ die Bedeutung von $R_{20}$ mit Ausnahme der Gruppen der Formel VII hat, und $R_{15}$, $R_{16}$, $R_{25}$ und $R_{28}$ die oben angegebene Bedeutung haben.

3. Neue Metallkomplexe gemäss Anspruch 1 und 2 der Formel I, worin B als einfach geladenes Anion beispielsweise ein solches einer aliphatischen oder aromatischen Carbonsäure, eines Phosphonsäure-Halbesters, einer Phosphinsäure, einer Phosphinigsäure oder eines Enols der Formel IX

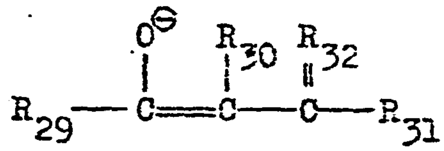

(IX),

worin

$R_{29}$ Alkyl, Alkenyl, Cycloalkyl, Aralkyl oder Aryl ist, und.

$R_{30}$ Wasserstoff, Alkyl, Alkenyl, Cycloalkyl, Aralkyl, Aryl oder Alkoxycarbonyl ist, oder

$R_{29}$ und $R_{30}$ zusammen gegebenenfalls substituiertes 1,4-Butadi-1,3-enylen oder 1,4-Butylen sind, und

$R_{31}$ gegebenenfalls substituiertes Alkyl, Alkenyl, Cyclc alkyl, Aralkyl, Aryl, Alkoxy oder gegebenenfalls substituiertes Amino bedeutet, und

$R_{32}$ Oxo oder gegebenenfalls substituiertes Imino ist, un

die übrigen Symbole die in Anspruch 1 und 2 gegebene Bedeutun haben.

4. Neue Metallkomplexe gemäss Anspruch 1 der Formel XI

$$[M^{q\oplus}]\,[L_1^{r\ominus}]_{q/r} \cdot mA \qquad (XIV),$$

worin

M $Mg^{2+}$, $VO^{2+}$, $Ca^{2+}$, $Mn^{2+}$, $Zn^{2+}$, $Co^{2+}$, $Ni^{2+}$ oder $Al^{3+}$ ist, und

q 2 oder 3 ist, und

$L_1$ eine t-zähnige Gruppe der Formel IVa, welche mindestens mit einer Gruppe der Formeln Va, Vb oder

VI substituiert ist, bedeutet, und

u 0, 1 oder 2 ist, und

t 3 ist, und

$R_{15}$ und $R_{16}$ unabhängig voneinander Wasserstoff, $C_1$-$C_{18}$ Alkyl, welches gegebenenfalls mit einer Gruppe der Formeln Va, Vb oder Vc substituiert sein kann; oder eine Gruppe Vc, oder eine p-ständige Gruppe der Formel VI, Halogen oder einen Rest $-OR_{24}$ bedeuten oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl, $C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-NR_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$

Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

r 1 oder 2 ist, und

m eine Zahl von 0 bis 3 ist, wobei die Summe $(3 \cdot q/r)$+ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aryl, $C_7$-$C_{20}$ Aralkyl, eine gegebenenfalls substituierte Aminoalkylgruppe

oder eine Piperidinylgruppe ist, und

$R_{13}$ Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden, und

$R_{14}$ Wasserstoff oder gegebenenfalls mit -OH substituiertes $C_1$-$C_{18}$ Alkyl bedeutet.

5. Neue Metallkomplexe gemäss Anspruch 1 der Formel

$$\left[M^{q\oplus}\right] \left[L_2^{r\ominus}\right]_{q/r} \cdot m A \qquad (XV),$$

worin

M $Mg^{2+}$, $Sr^{2+}$, $VO^{2+}$, $Ca^{2+}$, $Mn^{2+}$, $Zn^{2+}$, $Co^{2+}$, $Ni^{2+}$ ode $Al^{3+}$ ist, und

q 2 oder 3 ist, und

$L_2$ eine t-zähnige Gruppe der Formel IVb, welche mindestens mit einer Gruppe der Formeln Va, Vb oder \ substituiert ist, bedeutet, und

t 2 ist, und

$R_{15}$ und $R_{16}$ unabhängig voneinander Wasserstoff, $C_1$-$C_{18}$ Alkyl, welches gegebenenfalls mit einer Gruppe der Forme Va, Vb oder Vc substituiert sein kann; oder eine Gruppe Vc oder eine p-ständige Gruppe der Formel \ Halogen oder einen Rest der Formel $-OR_{24}$ bedeuten oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl,

$C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gegebenenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-NR_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl; oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel VI bedeutet, und

$R_{26}$ Wasserstoff, $C_1$-$C_{18}$ Alkyl, $C_3$-$C_6$ Alkenyl, Cyclohexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{17}$ Wasserstoff, $C_1$-$C_{18}$ Alkyl, Halogen, eine Gruppe $-OR_{24}$ oder eine Gruppe der Formel Vc, worin $R_{23}$, $R_{24}$ und $X_2$ die angegebene Bedeutung haben, bedeutet, und

r 1 ist, und

m eine Zahl von 0 bis 2 ist, wobei die Summe $(2 \cdot q)+m$ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aryl, $C_7$-$C_{20}$ Aralkyl, eine gegebenenfalls substituierte Aminoalkylgruppe oder eine Piperidinylgruppe ist, und

$R_{13}$ Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden, und

$R_{14}$ Wasserstoff oder gegebenenfalls mit -OH substituiertes $C_1$-$C_{18}$ Alkyl bedeutet.

6. Neue Metallkomplexe gemäss Anspruch 1 der Formel XVI

$$\left[M^{q\oplus}\right] \cdot \left[L_3^{r\ominus}\right]_{q/r} \cdot m\,A \qquad (XVI),$$

worin

M $Mg^{2+}$, $VO^{2+}$, $Ca^{2+}$, $Mn^{2+}$, $Zn^{2+}$, $Co^{2+}$, $Ni^{2+}$ oder $Al^{3+}$ ist, und

q 2 oder 3 ist, und

$L_3$ eine t-zähnige Gruppe der Formel IVc, welche mindestens mit einer Gruppe der Formeln Va, Vb oder VI substituiert ist, bedeutet, und

t 2 oder 4 ist, und

$R_{15}$ und $R_{16}$ unabhängig voneinander Wasserstoff, $C_1$-$C_{18}$ Alkyl, welches gegebenenfalls mit einer Gruppe der Formeln Va, Vb oder Vc substituiert sein kann oder eine Gruppe Vc, oder eine p-ständige Gruppe der Formel IV, Halogen oder einen Rest $-OR_{24}$ bedeuten, oder $R_{15}$ und $R_{16}$ zusammen einen 1,3-Butadien-1,4-diyl-Rest bilden, wobei

$R_5$ Wasserstoff, Methyl oder Phenyl bedeutet, und

Y Wasserstoff, Oxyl, $C_1$-$C_{12}$ Alkyl, $C_3$-$C_{12}$ Alkenyl, $C_3$-$C_6$ Alkinyl, $C_2$-$C_{21}$ Alkoxyalkyl, $C_7$-$C_9$ Aralkyl, 2,3-Epoxypropyl, eine aliphatische $C_1$-$C_4$ Acylgruppe oder eine der Gruppen $-CH_2COOR_6$, $-CH_2-CH(R_7)-OR_8$, $-COOR_9$ oder $-CONHR_9$ ist, worin

$R_6$ $C_1$-$C_{12}$ Alkyl, $C_3$-$C_6$ Alkenyl, Phenyl, $C_7$-$C_8$ Aralkyl oder Cyclohexyl ist und

$R_7$ Wasserstoff, Methyl oder Phenyl ist, und

$R_8$ Wasserstoff, eine aliphatische $C_1$-$C_{18}$ Acylgruppe, eine aromatische $C_7$ Acylgruppe, eine araliphatische $C_8$-$C_9$ Acylgruppe oder eine alicyclische $C_6$-$C_9$ Acylgruppe bedeutet, wobei der aromatische Teil gege

benenfalls mit Chlor, $C_1$-$C_4$ Alkyl, $C_1$-$C_8$ Alkoxy und/oder Hydroxy substituiert sein kann, und

$R_9$ $C_1$-$C_{12}$ Alkyl, Cyclohexyl, Phenyl oder Benzyl bedeutet, und

$X_1$ und $X_2$ unabhängig voneinander -O- oder $-\overset{|}{N}R_{25}$ sind, und

$R_{21}$ Wasserstoff oder $C_1$-$C_8$ Alkyl ist, und

$R_{22}$ Wasserstoff oder eine Gruppe $-X_1R_{26}$ bedeutet, und

Z -O- oder $-O-\overset{O}{\overset{\|}{C}}-$ ist, und

$R_{23}$ $C_1$-$C_{18}$ Alkyl, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, $C_7$-$C_{14}$ Alkylphenyl oder eine Gruppe der Formel VI bedeutet, worin Y und $R_{21}$ die angegebene Bedeutung haben, und

$R_{24}$ Wasserstoff, gegebenenfalls mit einer Gruppe der Formel Vc, worin $X_2$ und $R_{23}$ die angegebene Bedeutung hat, substituiertes $C_1$-$C_8$ Alkyl, $C_5$-$C_7$ Cycloalkyl, $C_6$-$C_{14}$ Aryl, $C_7$-$C_{14}$ Aralkyl, $C_2$-$C_{12}$ Alkenyl oder eine gegebenenfalls mit Chlor, $C_1$-$C_8$ Alkyl, $C_1$-$C_8$ Alkoxy, einer Gruppe Vc und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet und

$R_{25}$ Wasserstoff, $C_1$-$C_{18}$ Alkyl, das gegebenenfalls mit einer Gruppe der Formel Vc, worin $X_2$ und $R_{23}$ die angegebene Bedeutung hat, substituiert sein kann, $C_3$-$C_6$ Alkenyl, $C_3$-$C_4$ Alkinyl, $C_5$-$C_7$ Cycloalkyl, $C_6$-$C_{10}$ Aryl, $C_7$-$C_{14}$ Aralkyl, eine Gruppe der Formel $CH_2$-$CH(R_5)$-$OR_{26}$ oder eine Gruppe der Formel V bedeutet, und

$R_{26}$ Wasserstoff, $C_1$-$C_{18}$ Alkyl, $C_3$-$C_6$ Alkenyl, Cyclo-

hexyl, Benzyl oder eine gegebenenfalls mit Chlor, $C_1$-$C_8$ Alkyl, $C_1$-$C_8$ Alkoxy und/oder mit Hydroxy substituierte aliphatische $C_1$-$C_{18}$ Acylgruppe, aromatische $C_7$ Acylgruppe, araliphatische $C_8$-$C_9$ Acylgruppe oder alicyclische $C_6$-$C_9$ Acylgruppe bedeutet, und

$R_{18}$ eine Gruppe $-N(R_{25})R_{27}$ ist, und

$R_{19}$ Wasserstoff ist, oder

$R_{18}$ und $R_{19}$ zusammen =O oder =$NR_{27}$ sind, und

$R_{20}$ Wasserstoff, $C_7$-$C_{14}$ Aralkyl, $C_7$-$C_{14}$ Alkylphenyl, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder eine Gruppe der Formel VII oder falls $R_{18}$ und $R_{19}$ zusammen =O sind eine Gruppe der Formel Va, wobei $X_1$, $R_{21}$ und Y oben definierte Bedeutung haben, und

$R_{28}$ $C_1$-$C_{12}$ Alkylen, Butenylen, $C_6$-$C_{10}$ Arylen oder Diphenylen ist, bedeutet, und

$R_{27}$ Wasserstoff, $C_7$-$C_{14}$ Aralkyl, $C_7$-$C_{14}$ Alkylphenyl, eine Gruppe $-OR_{24}$, oder gegebenenfalls mit einer Gruppe der Formel Vc substituiertes $C_1$-$C_{18}$ Alkyl, $C_2$-$C_{12}$ Alkenyl, $C_5$-$C_{12}$ Cycloalkyl oder $C_6$-$C_{14}$ Aryl ist, oder eine Gruppe der Formel VI, oder eine Gruppe der Formel $-CH_2-CH(R_5)-OR_{26}$; oder falls $R_{20}$ nicht eine Gruppe der Formel VII ist; $R_{27}$ auch eine Gruppe der Formel VIIIa oder VIIIb bedeutet, und

r 1 oder 2 ist, und

m eine Zahl von 0 bis 2 ist, wobei die Summe $(t \cdot q/r)+m$ gleich der Koordinationszahl des Metallions M ist, und

A $H_2O$ oder ein Amin der Formel III ist, worin

$R_{12}$ gegebenenfalls mit -OH substituiertes $C_1$-$C_{18}$ Alkyl, $C_5$-$C_{12}$ Cycloalkyl, $C_6$-$C_{18}$ Aralkyl, $C_7$-$C_{20}$ Aralkyl, eine gegebenenfalls substituierte Aminoalkylgruppe oder eine Piperidinylgruppe ist, und

$R_{13}$ Wasserstoff oder gegebenenfalls mit -OH substituiertes $C_1$-$C_{18}$ Alkyl, $C_5$-$C_{12}$ Cycloalkyl, eine gegebenenfalls substituierte Aminoalkylgruppe oder eine Piperidinylgruppe bedeutet, oder

$R_{12}$ und $R_{13}$ zusammen mit dem N-Atom einen durch Alkylgruppen substituierten oder unsubstituierten Pyrrolidin-, Piperidin- oder Morpholinring bilden, und

$R_{14}$ Wasserstoff oder gegebenenfalls mit -OH substituiertes $C_1$-$C_{18}$ Alkyl bedeutet.

7. Verwendung der neuen Metallkomplexe gemäss Anspruch 1 als Lichtschutzmittel.

8. Das mit Hilfe der neuen Metallkomplexe gemäss Anspruch 1 geschützte organische Material.

9. Verfahren zum Stabilisieren von organischem Material, dadurch gekennzeichnet, dass man einen neuen Metallkomplex gemäss Anspruch 1 einarbeitet.

10. Höhermolekulare Verbindungen, dadurch gekennzeichnet, dass sie als wiederkehrende Struktureinheit einen Baustein der Zusammensetzung der Formel I gemäss Anspruch 1, worin r 2 ist, besitzen.

Europäisches Patentamt

# EUROPÄISCHER RECHERCHENBERICHT

Nummer der Anmeldung
EP 78 10 0325

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | CHEMICAL ABSTRACTS, 71, 49713q (1969) & IZV. NAUK AKAD. SSSR, Ser.Klim. 1969(3) 698-700. * Zusammenfassung * | 1-10 | C 07 D 211/00<br>C 07 D 401/00<br>C 07 D 491/00<br>C 08 K 5/00<br>C 08 L 23/06 |
| D | DE - A - 2 625 967 (CIBA-GEIGY) * Anspruch 1 * | 1-10 | |
| | GB - A - 1 365 319 (CIBA-GEIGY) * Seite 1 * | 1-10 | RECHERCHIERTE SACHGEBIETE (Int. Cl.²) |
| A | US - A - 3 879 396 (C.E.RAMEY) * Zusammenfassung; Spalte 9 * | 1-10 | C 08 K 5/00<br>C 08 K 5/34 |
| A | US - A - 4 001 181 (C.E.RAMEY) * Zusammenfassung; Spalte 10 * | 1-10 | |
| A | DE - A - 2 436 616 (CIBA-GEIGY) * Seite 26 * | 1-10 | KATEGORIE DER GENANNTEN DOKUMENTE |
| A | DE - A - 2 042 652 (AMERICAN CYANAMID) * Seite 20 * | 1-10 | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| A | US - A - 4 026 866 (M.RASBERGER) * Spalten 1 und 2 * | 1-10 | &: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 23-10-1978 | COQUELIN |